# EUROPEAN PATENT SPECIFICATION

(11) **EP 3 302 549 B1**
(45) Date of publication and mention of the grant of the patent: **03.07.2019**
(21) Application number: 16725104.0
(22) Date of filing: 23.05.2016
(51) Int. Cl.: A61K 39/395, A61K 31/496, A61K 31/40, A61P 35/02, C07K 16/28

(54) **COMBINATION THERAPY OF AN ANTI CD20 ANTIBODY WITH A BCL-2 INHIBITOR AND A MDM2 INHIBITOR**
KOMBINATIONSTHERAPIE AUS ANTI-CD20-ANTIKÖRPER MIT EINEM BCL-2-INHIBITOR UND EINEM MDM2-INHIBITOR
TRAITEMENT COMBINÉ D'UN ANTICORPS ANTI-CD20 AVEC UN INHIBITEUR DE BCL-2 ET UN INHIBITEUR DE MDM2

(30) Priority: 26.05.2015 EP 15169199
(43) Date of publication of application: 11.04.2018
(73) Proprietor: F. Hoffmann-La Roche AG, 4070 Basel (CH)
(72) Inventor: KLEIN, Christian, 8906 Bonstetten (CH); HERTING, Frank, 82377 Penzberg (DE); DANGL, Markus, 82402 Seeshaupt (DE)
(74) Representative: Winkler, Thomas
(86) International application number: PCT/EP2016/061517
(87) International publication number: WO 2016/188935

(56) References cited:
- WO-A1-2012/080389
- Roberts et al.: "325 Determination of Recommended Phase 2 Dose of ABT-199 (GDC-0199) Combined with Rituximab (R) in Patients with Relapsed / Refractory (R/R) Chronic Lymphocytic Leukemia (CLL)", 56th ASH Annual Meeting and Exposition , December 2014 (2014-12), XP002759664, Retrieved from the Internet: URL:https://ash.confex.com/ash/2014/webpro gram/Paper71378.html [retrieved on 2016-07-07]
- Ma et al.: "ABT-199 (GDC-0199) combined with rituximab (R) in patients (pts) with relapsed/refractory (R/R) chronic lymphocytic leukemia (CLL): Interim results of a phase 1b study.", 2014 ASCO Annual Meeting , June 2014 (2014-06), XP002759665, Retrieved from the Internet: URL:http://meetinglibrary.asco.org/content /132375-144 [retrieved on 2016-07-08]
- Flinn et al.: "4687 Preliminary Results of a Phase 1b Study (GP28331) Combining GDC-0199 (ABT-199) and Obinutuzumab in Patients with Relapsed/Refractory or Previously Untreated Chronic Lymphocytic Leukemia", 56th ASH Annual Meeting and Exposition , December 2014 (2014-12), XP002759666, Retrieved from the Internet: URL:https://ash.confex.com/ash/2014/webpro gram/Paper75779.html [retrieved on 2016-07-08]
- Herting et al.: "3915 Enhanced Activity of GA101, a Novel Type II, Glycoengineered CD20 Antibody, In Combination with Bendamustine or Fludarabine, and with the Bcl-2 Family Inhibitors ABT-737 or ABT-263", 52nd ASH Annual Meeting and Exposition , December 2010 (2010-12), XP002759667, Retrieved from the Internet: URL:https://ash.confex.com/ash/2010/webpro gram/Paper26413.html [retrieved on 2016-07-08]
- Herting et al.: "1780 Combination of the Glycoengineered Type II CD20 Antibody Obinutuzumab (GA101) and the MDM2 Selective Antagonist RG7388 Results in Superior Anti-Tumor Activity", 56th ASH Annual Meeting and Exposition , December 2014 (2014-12), XP002759668, Retrieved from the Internet: URL:https://ash.confex.com/ash/2014/webpro gram/Paper67783.html [retrieved on 2016-07-08]
- Dangl et al.: "Abstract 5505: Synergistic anticancer activity of clinical stage, non-genotoxic apoptosis inducing agents RG7388 (MDM2 antagonist) and ABT-199 (GDC-0199, BCL2 inhibitor) in p53 wild-type AML tumor models", AACR Annual Meeting 2014 , April 2014 (2014-04), XP002759669, Retrieved from the Internet: URL:http://cancerres.aacrjournals.org/cont ent/74/19_Supplement/5505 [retrieved on 2016-07-08]
- Rongqing et al.: "Activation of p53 By Novel MDM2 Antagonist RG7388 Overcomes AML Inherent and Acquired Resistance to Bcl-2 Inhibitor ABT-199 (GDC-0199)", 56th Annual Meeting of the American-Society-of-Hematology , December 2014 (2014-12), XP002759670, Retrieved from the Internet: URL:www.bloodjournal.org/content/124/21/21 62 [retrieved on 2016-07-08]

### SEQUENCE LISTING

<110> F. Hoffmann-La Roche AG
<120> Combination therapy
<130> P32901
<130> 31861
<150> EP 15169199.5
   <151> 2015-05-26
<160> 20
<170> PatentIn version 3.5
<210> 1
   <211> 112
   <212> PRT
   <213> Mus sp.
<220>
   <221> MISC_FEATURE
   <223> amino acid sequence of variable region of the heavy chain (VH) of murine monoclonal anti-CD20 antibody B-Ly1
<400> 1
   Gly Pro Glu Leu Val Lys Pro Gly Ala Ser Val Lys Ile Ser Cys Lys
   1               5                   10                  15
   Ala Ser Gly Tyr Ala Phe Ser Tyr Ser Trp Met Asn Trp Val Lys Leu
               20                  25                  30
   Arg Pro Gly Gln Gly Leu Glu Trp Ile Gly Arg Ile Phe Pro Gly Asp
           35                  40                  45
   Gly Asp Thr Asp Tyr Asn Gly Lys Phe Lys Gly Lys Ala Thr Leu Thr
       50                  55                  60
   Ala Asp Lys Ser Ser Asn Thr Ala Tyr Met Gln Leu Thr Ser Leu Thr
   65                  70                  75                  80
   Ser Val Asp Ser Ala Val Tyr Leu Cys Ala Arg Asn Val Phe Asp Gly
                   85                  90                  95
   Tyr Trp Leu Val Tyr Trp Gly Gln Gly Thr Leu Val Thr Val Ser Ala
               100                 105                 110
<210> 2
   <211> 103
   <212> PRT
   <213> Mus sp.
<220>
   <221> MISC_FEATURE
   <223> amino acid sequence of variable region of the light chain (VL) of murine monoclonal anti-CD20 antibody B-Ly1
<400> 2
   Asn Pro Val Thr Leu Gly Thr Ser Ala Ser Ile Ser Cys Arg Ser Ser
   1               5                   10                  15
   Lys Ser Leu Leu His Ser Asn Gly Ile Thr Tyr Leu Tyr Trp Tyr Leu
               20                  25                  30
   Gln Lys Pro Gly Gln Ser Pro Gln Leu Leu Ile Tyr Gln Met Ser Asn
           35                  40                  45
   Leu Val Ser Gly Val Pro Asp Arg Phe Ser Ser Ser Gly Ser Gly Thr
       50                  55                  60
   Asp Phe Thr Leu Arg Ile Ser Arg Val Glu Ala Glu Asp Val Gly Val
   65                  70                  75                  80
   Tyr Tyr Cys Ala Gln Asn Leu Glu Leu Pro Tyr Thr Phe Gly Gly Gly
                   85                  90                  95
   Thr Lys Leu Glu Ile Lys Arg
               100
<210> 3
   <211> 119
   <212> PRT
   <213> Artificial
<220>
   <223> amino acid sequences of variable region of the heavy chain (VH) of humanized B-Ly1 antibody (B-HH2)
<400> 3
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 4
   <211> 119
   <212> PRT
   <213> Artificial
<220>
   <223> amino acid sequences of variable region of the heavy chain (VH) of humanized B-Ly1 antibody (B-HH3)
<400> 4
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Leu Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 5
   <211> 119
   <212> PRT
   <213> Artificial
<220>
   <223> amino acid sequences of variable region of the heavy chain (VH) of humanized B-Ly1 antibody (B-HH4)
<400> 5
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 6
   <211> 119
   <212> PRT
   <213> Artificial
<220>
   <223> amino acid sequences of variable region of the heavy chain (VH) of humanized B-Ly1 antibody (B-HH5)
<400> 6
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Ser Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 7
   <211> 119
   <212> PRT
   <213> Artificial
<220>
   <223> amino acid sequences of variable region of the heavy chain (VH) of humanized B-Ly1 antibody (B-HH6)
<400> 7
   Gln Val Gln Leu Val Gln Ser Gly Ala Glu Val Lys Lys Pro Gly Ser
   1 5 10 15
   Ser Val Lys Val Ser Cys Lys Ala Ser Gly Tyr Ala Phe Ser Tyr Ser
   20 25 30
   Trp Ile Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 8
   <211> 119
   <212> PRT
   <213> Artificial
<220>
   <223> amino acid sequences of variable region of the heavy chain (VH) of humanized B-Ly1 antibody (B-HH7)
<400> 8
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Ile Ser Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 9
   <211> 119
   <212> PRT
   <213> Artificial
<220>
   <223> amino acid sequences of variable region of the heavy chain (VH) of humanized B-Ly1 antibody (B-HH8)
<400> 9
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 10
   <211> 119
   <212> PRT
   <213> Artificial
<220>
   <223> amino acid sequences of variable region of the heavy chain (VH) of humanized B-Ly1 antibody (B-HH9)
<400> 10
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
   115
<210> 11
   <211> 119
   <212> PRT
   <213> Artificial
<220>
   <223> amino acid sequences of variable region of the heavy chain (VH) of humanized B-Ly1 antibody (B-HL8)
<400> 11
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 12
   <211> 119
   <212> PRT
   <213> Artificial
<220>
   <223> amino acid sequences of variable region of the heavy chain (VH) of humanized B-Ly1 antibody (B-HL10)
<400> 12
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Ala Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 13
   <211> 119
   <212> PRT
   <213> Artificial
<220>
   <223> amino acid sequences of variable region of the heavy chain (VH) of humanized B-Ly1 antibody (B-HL11)
<400> 13
   Gln Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
   115
<210> 14
   <211> 119
   <212> PRT
   <213> Artificial
<220>
   <223> amino acid sequences of variable region of the heavy chain (VH) of humanized B-Ly1 antibody (B-HL12)
<400> 14
   Glu Val Gln Leu Val Glu Ser Gly Ala Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 15
   <211> 119
   <212> PRT
   <213> Artificial
<220>
   <223> amino acid sequences of variable region of the heavy chain (VH) of humanized B-Ly1 antibody (B-HL13)
<400> 15
   Glu Val Gln Leu Val Glu Ser Gly Gly Gly Val Val Lys Pro Gly Gly
   1               5                   10                  15
   Ser Leu Arg Leu Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 16
   <211> 119
   <212> PRT
   <213> Artificial
<220>
   <223> amino acid sequences of variable region of the heavy chain (VH) of humanized B-Ly1 antibody (B-HL14)
<400> 16
   Glu Val Gln Leu Val Glu Ser Gly Gly Gly Leu Lys Lys Pro Gly Gly
   1 5 10 15
   Ser Leu Arg Leu Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 17
   <211> 119
   <212> PRT
   <213> Artificial
<220>
   <223> amino acid sequences of variable region of the heavy chain (VH) of humanized B-Ly1 antibody (B-HL15)
<400> 17
   Glu Val Gln Leu Val Glu Ser Gly Gly Gly Leu Val Lys Pro Gly Ser
   1 5 10 15
   Ser Leu Arg Leu Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 18
   <211> 119
   <212> PRT
   <213> Artificial
<220>
   <223> amino acid sequences of variable region of the heavy chain (VH) of humanized B-Ly1 antibody (B-HL16)
<400> 18
   Glu Val Gln Leu Val Glu Ser Gly Gly Gly Leu Val Lys Pro Gly Gly
   1 5 10 15
   Ser Leu Arg Val Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 19
   <211> 119
   <212> PRT
   <213> Artificial
<220>
   <223> amino acid sequences of variable region of the heavy chain (VH) of humanized B-Ly1 antibody (B-HL17)
<400> 19
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 20
   <211> 115
   <212> PRT
   <213> Artificial
<220>
   <223> amino acid sequences of variable region of the light chain (VL) of humanized B-Ly1 antibody B-KV1
<400> 20
   Asp Ile Val Met Thr Gln Thr Pro Leu Ser Leu Pro Val Thr Pro Gly
   1               5                   10                  15
   Glu Pro Ala Ser Ile Ser Cys Arg Ser Ser Lys Ser Leu Leu His Ser
               20                  25                  30
   Asn Gly Ile Thr Tyr Leu Tyr Trp Tyr Leu Gln Lys Pro Gly Gln Ser
           35                  40                  45
   Pro Gln Leu Leu Ile Tyr Gln Met Ser Asn Leu Val Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ala Gln Asn
                   85                  90                  95
   Leu Glu Leu Pro Tyr Thr Phe Gly Gly Gly Thr Lys Val Glu Ile Lys
               100                 105                 110
   Arg Thr Val
           115

## Description

The present invention is directed to the combination therapy of an anti CD20 antibody with a Bcl-2 inhibitor and a MDM2 inhibitor for the treatment of cancer.

### Background of the Invention

### Afucosylated antibodies

Cell-mediated effector functions of monoclonal antibodies can be enhanced by engineering their oligosaccharide component as described in Umaña, P., et al., Nature Biotechnol. 17 (1999) 176-180; and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M.R., et al., Glycobiology 5 (1995) 813-822; Jefferis, R., et al., Immunol. Rev. 163 (1998) 59-76; Wright, A., and Morrison, S.L., Trends Biotechnol. 15 (1997) 26-32). Umaña, P., et al.. Nature Biotechnol. 17 (1999) 176-180 and WO 99/154342 showed that overexpression in Chinese hamster ovary (CHO) cells of β(1,4)-N-acetylglucosaminyltransferase III ("GnTIII"), a glycosyltransferase catalyzing the formation of bisected oligosaccharides, significantly increases the in vitro ADCC activity of antibodies. Alterations in the composition of the N297 carbohydrate or its elimination affect also binding to Fc binding to FcγR and C1 q (Umaña, P., et al., Nature Biotechnol. 17 (1999) 176-180; Davies, J., et al., Biotechnol. Bioeng. 74 (2001) 288-294; Mimura, Y., et al., J. Biol. Chem. 276 (2001) 45539-45547; Radaev, S., et al., J. Biol. Chem. 276 (2001) 16478-16483; Shields, R.L., et al., J. Biol. Chem. 276 (2001) 6591-6604; Shields, R.L., et al., J. Biol. Chem. 277 (2002) 26733-26740; Simmons, L.C., et al., J. Immunol. Methods 263 (2002) 133-147). Studies discussing the activities of afucosylated and fucosylated antibodies, including anti-CD20 antibodies, have been reported (e.g., Iida, S., et al., Clin. Cancer Res. 12 (2006) 2879-2887; Natsume, A., et al., J. Immunol. Methods 306 (2005) 93-103; Satoh, M., et al., Expert Opin. Biol. Ther. 6 (2006) 1161-1173; Kanda, Y., et al., Biotechnol. Bioeng. 94 (2006) 680-688; Davies, J., et al., Biotechnol. Bioeng. 74 (2001) 288-294.

### CD20 and anti CD20 antibodies

The CD20 molecule (also called human B-lymphocyte-restricted differentiation antigen or Bp35) is a hydrophobic transmembrane protein located on pre-B and mature B lymphocytes that has been described extensively (Valentine, M.A., et al., J. Biol. Chem. 264 (1989) 11282-11287; and Einfeld, D.A., et al., EMBO J. 7 (1988) 711-717; Tedder, T.F., et al., Proc. Natl. Acad. Sci. U.S.A. 85 (1988) 208-212; Stamenkovic, I., et al., J. Exp. Med. 167 (1988) 1975-1980; Tedder, T.F., et al., J. Immunol. 142 (1989) 2560-2568). CD20 is expressed on greater than 90 % of B cell non-Hodgkin's lymphomas (NHL) (Anderson, K.C., et al., Blood 63 (1984) 1424-1433) but is not found on hematopoietic stem cells, pro-B cells, normal plasma cells, or other normal tissues (Tedder, T.F., et al., J, Immunol. 135 (1985) 973- 979).

There exist two different types of anti-CD20 antibodies differing significantly in their mode of CD20 binding and biological activities (Cragg, M.S., et al., Blood 103 (2004) 2738-2743; and Cragg, M.S., et al., Blood 101 (2003) 1045-1052). Type I antibodies, as, e.g., rituximab (a non-afucosylated antibody with an amount of fucose of 85 % or higher), ofatumumab, veltuzumab, ocrelizumab are potent in complement mediated cytotoxicity.

Type II antibodies, as e.g. Tositumomab (B1), 11B8, AT80 or humanized B-Ly1 antibodies, effectively initiate target cell death via caspase-independent cell death induction with concomitant phosphatidylserine exposure.

Anti-CD20 antibodies have been used in combination with Bcl-2 inhibitors (Roberts et al., BLOOD, 124(21), Abstract 325, 2014*;* Ma et al., J. Clin.Onc., 32(15, Suppl.), Abstract 7013, 2014*;* Flinn et al., BLOOD, 124(21), Abstract 4687, 2014*,* Herting et al.,BLOOD, 116(21), Abstract 3915, 2010*).*

Anti-CD20 antibodies have been used in combination with MDM2 inhibitors (Herting et al.,BLOOD, 124(21), Abstract 1780*;* WO2012080389).

### MDM2

p53 is a tumor suppressor protein that plays a central role in protection against development of cancer. It guards cellular integrity and prevents the propagation of permanently damaged clones of cells by the induction of growth arrest or apoptosis. At the molecular level, p53 is a transcription factor that can activate a panel of genes implicated in the regulation of cell cycle and apoptosis. p53 is a potent cell cycle inhibitor which is tightly regulated by MDM2 at the cellular level. MDM2 and p53 form a feedback control loop. MDM2 can bind p53 and inhibit its ability to transactivate p53-regulated genes. In addition, MDM2 mediates the ubiquitin-dependent degradation of p53. p53 can activate the expression of the MDM2 gene, thus raising the cellular level of MDM2 protein. This feedback control loop insures that both MDM2 and p53 are kept at a low level in normal proliferating cells. MDM2 is also a cofactor for E2F, which plays a central role in cell cycle regulation.

The ratio of MDM2 to p53 (E2F) is dysregulated in many cancers. Frequently occurring molecular defects in the p16INK4/p19ARF locus, for instance, have been shown to affect MDM2 protein degradation. Inhibition of MDM2-p53 interaction in tumor cells with wild-type p53 should lead to accumulation of p53, cell cycle arrest and/or apoptosis. MDM2 antagonists, therefore, can offer a novel approach to cancer therapy as single agents or in combination with a broad spectrum of other antitumor therapies. The feasibility of this strategy has been shown by the use of different macromolecular tools for inhibition of MDM2-p53 interaction (e.g. antibodies, antisense oligonucleotides, peptides). MDM2 also binds E2F through a conserved binding region as p53 and activates E2F-dependent transcription of cyclin A, suggesting that MDM2 antagonists might have effects in p53 mutant cells.

### Bcl-2 and Bcl-2 inhibitors

Bcl-2 proteins play a role in many diseases, particularly in cancer, leukemia, immune and autoimmune diseases. Bcl-2 proteins are said to be involved in bladder cancer, brain cancer, breast cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, prostate cancer, small cell lung cancer, spleen cancer. Overexpression of Bcl-2 proteins correlate with resistance to chemotherapy, clinical outcome, disease progression, overall prognosis or a combination thereof in various cancers and disorders of the immune system.

Bcl-2 inhibitors and MDM-2 inhibitors have been used in combination (Dangl et al., CANC.RES., 74(19), Abstract 5505, 2014*;* Rongqing et al., BLOOD, 124(21), Abstract 2162, 2014*).*

### Summary of the Invention

We have now found out that the combination of type I anti-CD20 antibody, or an afucosylated, type II anti-CD20 antibody with a Bcl-2 inhibitor and a MDM2 inhibitor showed significantly enhanced antiproliferative effects. Surprisingly, this combination is more than additive, i.e. highly synergistic.

Embodiments of the invention are defined in the appended claims.

One aspect of the disclosure is an afucosylated anti-CD20 antibody with an amount of fucose of 60% or less of the total amount of oligosaccharides (sugars) at Asn297, for the treatment of cancer in combination with a Bcl-2 inhibitor and a MDM2 inhibitor.

Another aspect of the disclosure is the use of an afucosylated anti-CD20 antibody with an amount of fucose of 60% or less of the total amount of oligosaccharides (sugars) at Asn297, for the manufacture of a medicament for the treatment of cancer in combination with a Bcl-2 inhibitor and a MDM2 inhibitor.

Another aspect of the disclosure is a method of treatment of patient suffering from cancer by administering an afucosylated anti-CD20 antibody with an amount of fucose of 60% or less of the total amount of oligosaccharides (sugars) at Asn297, in combination with a Bcl-2 inhibitor and a MDM2 inhibitor, to a patient in the need of such treatment.

In one embodiment, the amount of fucose is between 40% and 60% of the total amount of oligosaccharides (sugars) at Asn297. In another embodiment, the amount of fucose is 0% of the total amount of oligosaccharides (sugars) at Asn297.

In one aspect, the type I anti-CD20 antibody is rituximab. In one embodiment, the afucosylated anti-CD20 antibody is an IgG1 antibody. In another embodiment, said cancer is a CD20 expressing cancer, preferably a lymphoma or lymphocytic leukemia. In one embodiment said afucosylated anti-CD20 antibody is humanized B-Ly1 antibody. In another embodiment, said afucosylated antibody is a type II anti-CD20 antibody. In a preferred embodiment, said afucosylated antibody is obinutuzumab.

In one aspect, said Bcl-2 inhibitor is a compound selected from the compounds described in WO2010/138588. Methods of producing said Bcl-2 inhibitors are also disclosed in WO2010/138588.

According to the invention, the Bcl-2 inhibitor is 4-(4-{[2-(4-chlorophenyl)-4,4-dimethylcyclohex-1-en-1-yl]methyl}piperazin-1-yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide as in the formula below or a salt, ester or polymorph thereof. Example 5 of WO2010/138588 describes methods for preparation of said compound.

Said Bcl-2 inhibitor is also depicted in the following formula: 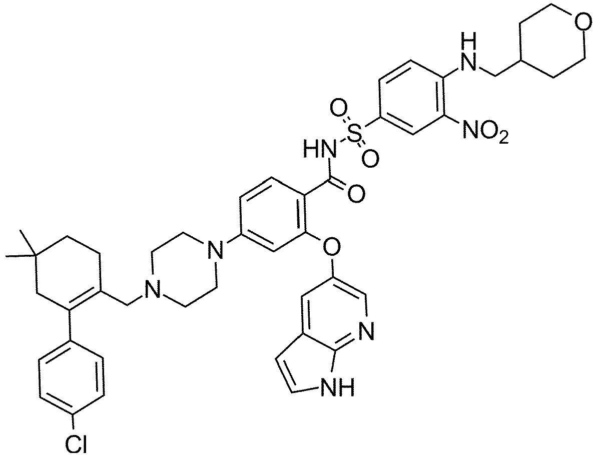 Said compound is also named ABT-199 (GDC-0199 or Venetoclax).

In one aspect of the disclosure, said MDM2 inhibitor is a compound selected from the compounds described in WO2011/098398. Methods of producing said MDM2 inhibitors are also disclosed in WO2011/098398.

According to the invention, the MDM2 inhibitor is 4-{[(2R,3S,4R,5S)-4-(4-Chloro-2-fluorophenyl)-3-(3-chloro-2-fluoro-phenyl)-4-cyano-5-(2,2-dimethyl-propyl)-pyrrolidine-2-carbonyl]-amino }-3-methoxy-benzoic acid as in the formula below or a salt, ester or polymorph thereof. Example 448 of WO2011/098398 describes a method for preparation of said compound. 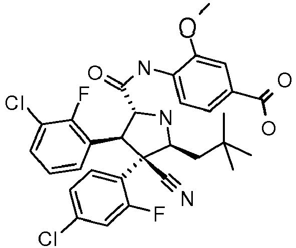 Molecular Weight =616.4973
Molecular Formula =C31 H29CI2F2N3O4

In one aspect of the disclosure, said type I anti-CD20 antibody is rituximab, said afucosylated anti-CD20 antibody is humanized B-Ly1 antibody, preferably obinutuzumab, and Bcl-2 inhibitor, wherein the Bcl-2 inhibitor is selected from the compounds described in WO 2010/138588 and a MDM2 inhibitor selected from the compounds described in WO2011/098398. Said MDM2 inhibitor preferably is a compound according to formula I or according to formula 1-1 as disclosed in the present specification above. Preferably, the MDM2 inhibitor is 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino }-3-methoxy-benzoic acid or a salt thereof, and said cancer is a CD20 expressing cancer, in one aspect a lymphoma or lymphocytic leukemia.

In one embodiment, the afucosylated anti-CD20 antibody binds CD20 with an KD of 10⁻⁸ M to 10⁻¹³ M.

One aspect of the disclosure is a pharmaceutical composition comprising a combination of a type I anti-CD20 antibody (in one embodiment rituximab) or an afucosylated anti-CD20 antibody with an amount of fucose of 60% or less of the total amount of oligosaccharides (sugars) at Asn297, (in one aspect an afucosylated humanized B-Ly1 antibody or preferably obinutuzumab), and a Bcl-2 inhibitor, wherein the Bcl-2 inhibitor is selected from the compounds described in WO 2010/138588 and a MDM2 inhibitor, wherein the MDM2 inhibitor is selected from the compounds described in WO2011/098398. Said MDM2 inhibitor preferably is a compound according to formula I or according to formula 1-1 as disclosed in the present specification above. Preferably, the MDM2 inhibitor is 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid or a salt thereof is 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid or a salt thereof for the treatment of cancer.

### Description of the Figures

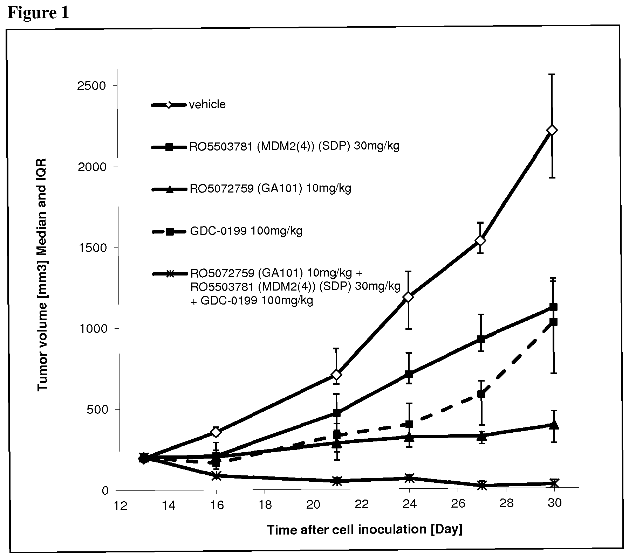

Figure 1: Tumor volume development until day 29 and IQR values (example 1)

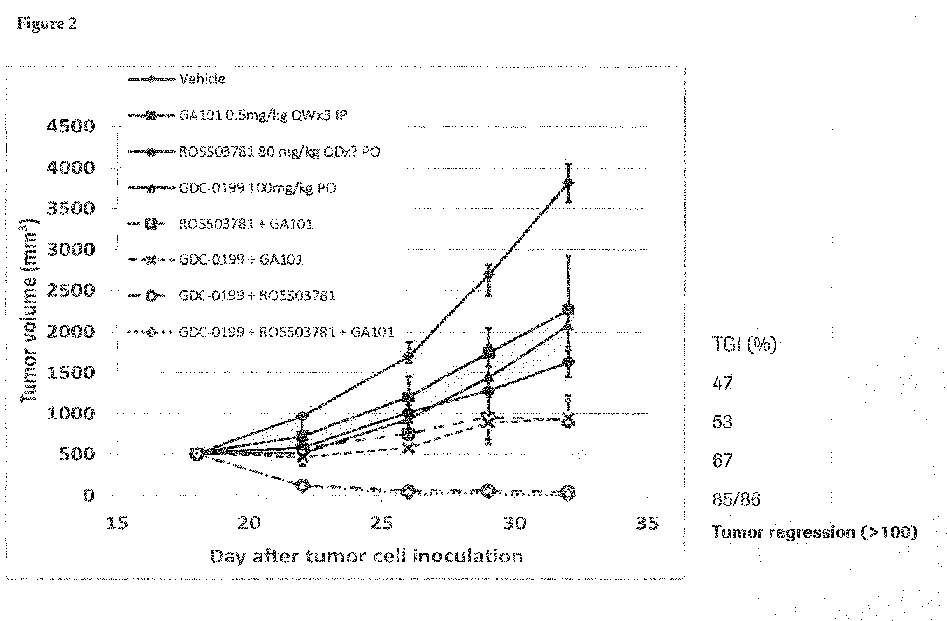

Figure 2: Tumor volume development until day 32 and TGI values (example 2)

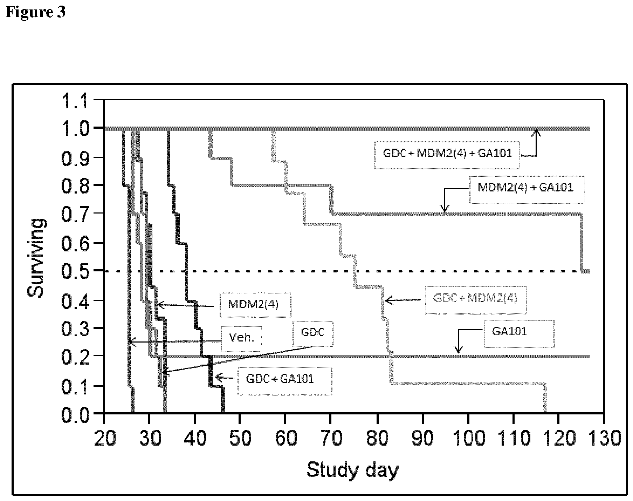

Figure 3: Time-to- event analysis until day 125 (example 2)

### Detailed Description of the Invention

The invention comprises a an afucosylated anti-CD20 antibody of IgG1 or IgG3 isotype with an amount of fucose of 60% or less of the total amount of oligosaccharides (sugars) at Asn297, for the treatment of cancer in combination with a Bcl-2 inhibitor and a MDM2 inhibitor, as defined by the claims.

The invention comprises the use of an afucosylated anti-CD20 antibody of IgG1 or IgG3 isotype with an amount of fucose of 60% or less of the total amount of oligosaccharides (sugars) at Asn297, for the manufacture of a medicament for the treatment of cancer in combination with a Bcl-2 inhibitor and a MDM2 inhibitor, as defined by the claims.

In one embodiment, the amount of fucose is between 40% and 60% of the total amount of oligosaccharides (sugars) at Asn297.

The term "antibody" encompasses the various forms of antibodies including but not being limited to whole antibodies, human antibodies, humanized antibodies and genetically engineered antibodies like monoclonal antibodies, chimeric antibodies or recombinant antibodies as well as fragments of such antibodies as long as the characteristic properties according to the invention are retained. The terms "monoclonal antibody" or "monoclonal antibody composition" as used herein refer to a preparation of antibody molecules of a single amino acid composition. Accordingly, the term "human monoclonal antibody" refers to antibodies displaying a single binding specificity which have variable and constant regions derived from human germline immunoglobulin sequences. Human monoclonal antibodies are produced by a hybridoma which includes a B cell obtained from a transgenic non-human animal, e.g. a transgenic mouse, having a genome comprising a human heavy chain transgene and a light human chain transgene fused to an immortalized cell.

The term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad Sci. USA 81 (1984) 6851-6855; US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L. et al., Nature 332 (1988) 323-327; and Neuberger, M.S. et al., Nature 314 (1985) 268-270. Particularly preferred CDRs correspond to those representing sequences recognizing the antigens noted above for chimeric and bi-or multispecific antibodies.

The term "human antibody", as used herein, is intended to include antibodies having variable and constant regions derived from human germline immunoglobulin sequences. Human antibodies are well-known in the state of the art (van Dijk, M.A., and van de Winkel, J.G., Curr. Opin. in Chem. Biol. 5 (2001) 368-374). Based on such technology, human antibodies against a great variety of targets can be produced. Examples of human antibodies are for example described in Kellermann, S.A., et al., Curr Opin Biotechnol. 13 (2002) 593-597.

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions derived from human germline immunoglobulin sequences in a rearranged form. The recombinant human antibodies according to the invention have been subjected to in vivo somatic hypermutation. Thus, the amino acid sequences of the VH and VL regions of the recombinant antibodies are sequences that, while derived from and related to human germline VH and VL sequences, may not naturally exist within the human antibody germline repertoire in vivo.

The term "bi- or multispecific antibody" as used herein relates to monoclonal antibodies that have binding specificities for at least two different sites. In certain embodiments, one of the binding specificities is for CD20 and the other is for any other antigen. In certain embodiments, bispecific antibodies may bind to two different epitopes of CD20. Bispecific antibodies may also be used to localize cytotoxic agents to cells which express CD20. Bispecific antibodies can be prepared as full length antibodies or antibody fragments.

As used herein, the term "binding" or "specifically binding" refers to the binding of the antibody to an epitope of the tumor antigen in an in vitro assay, preferably in an plasmon resonance assay (BIAcore, GE-Healthcare Uppsala, Sweden) with purified wild-type antigen. The affinity of the binding is defined by the terms ka (rate constant for the association of the antibody from the antibody/antigen complex), k_{D} (dissociation constant), and K_{D} (k_{D}/ka). Binding or specifically binding means a binding affinity (K_{D}) of 10⁻⁸ M or less, preferably 10⁻⁸ M to 10⁻¹³ M (in one embodiment 10⁻⁹ M to 10⁻¹³ M). Thus, an afucosylated antibody according to the invention is specifically binding to the tumor antigen with a binding affinity (K_{D}) of 10⁻⁸ mol/l or less, preferably 10⁻⁸ M to 10⁻¹³ M (in one embodiment 10⁻⁹ M to 10^{- 13} M).

The term "nucleic acid molecule", as used herein, is intended to include DNA molecules and RNA molecules. A nucleic acid molecule may be single-stranded or double-stranded, but preferably is double-stranded DNA.

The "constant domains" are not involved directly in binding the antibody to an antigen but are involved in the effector functions (ADCC, complement binding, and CDC).

The "variable region" (variable region of a light chain (VL), variable region of a heavy chain (VH)) as used herein denotes each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain the antigen binding site.

The terms "hypervariable region" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. Especially, CDR3 of the heavy chain is the region which contributes most to antigen binding. CDR and FR regions are determined according to the standard definition of Kabat, et al., Sequences of Proteins of Immunological Interest, 5th ed., Public Health Service, National Institutes of Health, Bethesda, MD (1991), and/or those residues from a "hypervariable loop".

The term "afucosylated antibody" refers to an antibody of IgG1 or IgG3 isotype (preferably of IgG1 isotype) with an altered pattern of glycosylation in the Fc region at Asn297 having a reduced level of fucose residues. Glycosylation of human IgG1 or IgG3 occurs at Asn297 as core fucosylated bianntennary complex oligosaccharide glycosylation terminated with up to 2 Gal residues. These structures are designated as G0, G1 (α1,6 or α1,3) or G2 glycan residues, depending from the amount of terminal Gal residues (Raju, T.S., BioProcess Int. 1 (2003) 44-53). CHO type glycosylation of antibody Fc parts is e.g. described by Routier, F.H., Glycoconjugate J. 14 (1997) 201-207. Antibodies which are recombinantely expressed in non glycomodified CHO host cells usually are fucosylated at Asn297 in an amount of at least 85%. It should be understood that the term an afucosylated antibody as used herein includes an antibody having no fucose in its glycosylation pattern. It is commonly known that typical glycosylated residue position in an antibody is the asparagine at position 297 according to the EU numbering system ("Asn297").

The "EU numbering system" or "EU index" is generally used when referring to a residue in an immunoglobulin heavy chain constant region (e.g., the EU index reported in Kabat et al., Sequences of Proteins of Immunological Interest, 5th ed., Public Health Service, National Institutes of Health, Bethesda, MD (1991)). Thus an afucosylated antibody according to the invention means an antibody of IgG1 or IgG3 isotype (preferably of IgG1 isotype) wherein the amount of fucose is 60% or less of the total amount of oligosaccharides (sugars) at Asn297 (which means that at least 40% or more of the oligosaccharides of the Fc region at Asn297 are afucosylated). In one embodiment the amount of fucose is between 40% and 60% of the oligosaccharides of the Fc region at Asn297. In another embodiment the amount of fucose is 50% or less, and in still another embodiment the amount of fucose is 30% or less of the oligosaccharides of the Fc region at Asn297. According to the invention "amount of fucose" means the amount of said oligosaccharide (fucose) within the oligosaccharide (sugar) chain at Asn297, related to the sum of all oligosaccharides (sugars) attached to Asn 297 (e. g. complex, hybrid and high mannose structures) measured by MALDI-TOF mass spectrometry and calculated as average value (for a detailed procedure to determine the amount of fucose, see e.g. WO 2008/077546). Furthermore in one embodiment, the oligosaccharides of the Fc region are bisected. The afucosylated antibody according to the invention can be expressed in a glycomodified host cell engineered to express at least one nucleic acid encoding a polypeptide having GnTIII activity in an amount sufficient to partially fucosylate the oligosaccharides in the Fc region. In one embodiment, the polypeptide having GnTIII activity is a fusion polypeptide. Alternatively α1,6-fucosyltransferase activity of the host cell can be decreased or eliminated according to US 6,946,292 to generate glycomodified host cells. The amount of antibody fucosylation can be predetermined e.g. either by fermentation conditions (e.g. fermentation time) or by combination of at least two antibodies with different fucosylation amount. Such afucosylated antibodies and respective glycoengineering methods are described in WO 2005/044859, WO 2004/065540, WO 2007/031875, Umana, P., et al., Nature Biotechnol. 17 (1999) 176-180, WO 99/154342, WO 2005/018572, WO 2006/116260, WO 2006/114700, WO 2005/011735, WO 2005/027966, WO 97/028267, US 2006/0134709, US 2005/0054048, US 2005/0152894, WO 2003/035835, WO 2000/061739. These glycoengineered antibodies have an increased ADCC. Other glycoengineering methods yielding afucosylated antibodies according to the invention are described e.g. in Niwa, R.. et al., J. Immunol. Methods 306 (2005) 151-160; Shinkawa, T., et al., J. Biol. Chem, 278 (2003) 3466-3473; WO 03/055993 or US 2005/0249722.

Thus one aspect of the invention relates to an afucosylated anti-CD20 antibody of IgG1 or IgG3 isotype (preferably of IgG1 isotype) specifically binding to CD20 with an amount of fucose of 60% or less of the total amount of oligosaccharides (sugars) at Asn297, for the treatment of cancer in combination with a Bcl-2 inhibitor and a MDM2 inhibitor, as defined by the claims. In another aspect of the invention is the use of an afucosylated anti-CD20 antibody of IgG1 or IgG3 isotype (preferably of IgG1 isotype) specifically binding to CD20 with an amount of fucose of 60% or less of the total amount of oligosaccharides (sugars) at Asn297, for the manufacture of a medicament for the treatment of cancer in combination with a Bcl-2 inhibitor and a MDM2 inhibitor, as defined by the claims. In one embodiment, the amount of fucose is between 60% and 20% of the total amount of oligosaccharides (sugars) at Asn297. In one embodiment the amount of fucose is between 60% and 40% of the total amount of oligosaccharides (sugars) at Asn297. In one embodiment, the amount of fucose is between 0% of the total amount of oligosaccharides (sugars) at Asn297.

CD20 (also known as B-lymphocyte antigen CD20, B-lymphocyte surface antigen B1, Leu-16, Bp35, BM5, and LF5; the sequence is characterized by the SwissProt database entry P11836) is a hydrophobic transmembrane protein with a molecular weight of approximately 35 kD located on pre-B and mature B lymphocytes (Valentine, M.A. et al., J. Biol. Chem. 264 (1989) 11282-11287; Tedder, T.F., et al., Proc. Natl. Acad. Sci. U.S.A. 85 (1988) 208-212; Stamenkovic, I., et al., J. Exp. Med. 167 (1988) 1975-1980; Einfeld, D.A., et al., EMBO J. 7 (1988) 711-717; Tedder, T.F., et al., J. Immunol. 142 (1989) 2560-2568). The corresponding human gene is Membrane-spanning 4-domains, subfamily A, member 1, also known as MS4A1. This gene encodes a member of the membrane-spanning 4A gene family. Members of this nascent protein family are characterized by common structural features and similar intron/exon splice boundaries and display unique expression patterns among hematopoietic cells and nonlymphoid tissues. This gene encodes the B-lymphocyte surface molecule which plays a role in the development and differentiation of B-cells into plasma cells. This family member is localized to 11q12, among a cluster of family members. Alternative splicing of this gene results in two transcript variants which encode the same protein.

The terms "CD20" and "CD20 antigen" are used interchangeably herein, and include any variants, isoforms and species homologs of human CD20 which are naturally expressed by cells or are expressed on cells transfected with the CD20 gene. Binding of an antibody of the invention to the CD20 antigen mediate the killing of cells expressing CD20 (e.g., a tumor cell) by inactivating CD20. The killing of the cells expressing CD20 may occur by one or more of the following mechanisms: Cell death/apoptosis induction, ADCC and CDC.

Synonyms of CD20, as recognized in the art, include B-lymphocyte antigen CD20, B-lymphocyte surface antigen B1, Leu-16, Bp35, BM5, and LF5.

The term "anti-CD20 antibody" according to the invention is an antibody that binds specifically to CD20 antigen. Depending on binding properties and biological activities of anti-CD20 antibodies to the CD20 antigen, two types of anti-CD20 antibodies (type I and type II anti-CD20 antibodies) can be distinguished according to Cragg, M.S., et al., Blood 103 (2004) 2738-2743; and Cragg, M.S., et al., Blood 101 (2003) 1045-1052, see Table 1.

**Table 1: Properties of type I and type II anti-CD20 antibodies**

| **type I anti-CD20 antibodies** | **type II anti-CD20 antibodies** |
|---|---|
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity (if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

Examples of type II anti-CD20 antibodies include e.g. humanized B-Ly1 antibody IgG1 (a chimeric humanized IgG1 antibody as disclosed in WO 2005/044859), 11B8 IgG1 (as disclosed in WO 2004/035607), and AT80 IgG1. Typically type II anti-CD20 antibodies of the IgG1 isotype show characteristic CDC properties. Type II anti-CD20 antibodies have a decreased CDC (if IgG1 isotype) compared to type I antibodies of the IgG1 isotype.

Examples of type I anti-CD20 antibodies include e.g. rituximab, HI47 IgG3 (ECACC, hybridoma), 2C6 IgG1 (as disclosed in WO 2005/103081), 2F2 IgG1 (as disclosed and WO 2004/035607 and WO 2005/103081) and 2H7 IgG1 (as disclosed in WO 2004/056312).

The afucosylated anti-CD20 antibody for use according to the invention is an afucosylated humanized B-Ly1 antibody.

The afucosylated anti-CD20 antibodies for use according to the invention have an increased antibody dependent cellular cytotoxicity (ADCC) unlike anti-CD20 antibodies having no reduced fucose.

By "afucosylated anti-CD20 antibody with increased antibody dependent cellular cytotoxicity (ADCC)" is meant an afucosylated anti-CD20 antibody, as that term is defined herein, having increased ADCC as determined by any suitable method known to those of ordinary skill in the art. One accepted in vitro ADCC assay is as follows:
1) the assay uses target cells that are known to express the target antigen recognized by the antigen-binding region of the antibody;
2) the assay uses human peripheral blood mononuclear cells (PBMCs), isolated from blood of a randomly chosen healthy donor, as effector cells;
3) the assay is carried out according to following protocol:
   i) the PBMCs are isolated using standard density centrifugation procedures and are suspended at 5 x 10⁶ cells/ml in RPMI cell culture medium;
   ii) the target cells are grown by standard tissue culture methods, harvested from the exponential growth phase with a viability higher than 90%, washed in RPMI cell culture medium, labeled with 100 micro-Curies of ⁵¹Cr, washed twice with cell culture medium, and resuspended in cell culture medium at a density of 10⁵ cells/ml;
   iii) 100 microliters of the final target cell suspension above are transferred to each well of a 96-well microtiter plate;
   iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;
   v) for the maximum release (MR) controls, 3 additional wells in the plate containing the labeled target cells, receive 50 microliters of a 2% (VN) aqueous solution of non-ionic detergent (Nonidet, Sigma, St. Louis), instead of the antibody solution (point iv above);
   vi) for the spontaneous release (SR) controls, 3 additional wells in the plate containing the labeled target cells, receive 50 microliters of RPMI cell culture medium instead of the antibody solution (point iv above);
   vii) the 96-well microtiter plate is then centrifuged at 50 x g for 1 minute and incubated for 1 hour at 4°C;
   viii) 50 microliters of the PBMC suspension (point i above) are added to each well to yield an effector:target cell ratio of 25: 1 and the plates are placed in an incubator under 5% CO₂ atmosphere at 37 °C for 4 hours;
   ix) the cell-free supernatant from each well is harvested and the experimentally released radioactivity (ER) is quantified using a gamma counter;
   x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point v above), and SR is the average radioactivity quantified (see point ix above) for the SR controls (see point vi above);
4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. The increase in ADCC is relative to the ADCC, measured with the above assay, mediated by the same antibody, produced by the same type of host cells, using the same standard production, purification, formulation and storage methods, which are known to those skilled in the art, but that has not been produced by host cells engineered to overexpress GnTIII.

Said "increased ADCC" can be obtained by glycoengineering of said antibodies, that means enhance said natural, cell-mediated effector functions of monoclonal antibodies by engineering their oligosaccharide component as described in Umana, P., et al., Nature Biotechnol. 17 (1999) 176-180 and US 6,602,684.

The term "complement-dependent cytotoxicity (CDC)" refers to lysis of human tumor target cells by the antibody in the presence of complement. CDC is measured preferably by the treatment of a preparation of CD20 expressing cells with an anti-CD20 antibody in the presence of complement. CDC is found if the antibody induces at a concentration of 100 nM the lysis (cell death) of 20% or more of the tumor cells after 4 hours. The assay is performed preferably with ⁵¹Cr or Eu labeled tumor cells and measurement of released ⁵¹Cr or Eu. Controls include the incubation of the tumor target cells with complement but without the antibody.

The "rituximab" antibody (example of a type I anti-CD20 antibody) is a genetically engineered chimeric human gamma 1 murine constant domain containing monoclonal antibody directed against the human CD20 antigen.

The "rituximab" antibody (example of a type I anti-CD20 antibody) is a genetically engineered chimeric human gamma 1 murine constant domain containing monoclonal antibody directed against the human CD20 antigen. This chimeric antibody contains human gamma 1 constant domains and is identified by the name "C2B8" in US 5,736,137 (Anderson et. al.) issued on April 17, 1998, assigned to IDEC Pharmaceuticals Corporation. Rituximab is approved for the treatment of patients with relapsed or refracting low-grade or follicular, CD20 positive, B cell non-Hodgkin's lymphoma. In vitro mechanism of action studies have shown that rituximab exhibits human complement-dependent cytotoxicity (CDC) (Reff, M.E., et. al., Blood 83 (1994) 435-445). Additionally, it exhibits significant activity in assays that measure antibody-dependent cellular cytotoxicity (ADCC). Rituximab is not afucosylated.

**Table 2**

| Antibody | Amount of fucose |
|---|---|
| Rituximab (non-afucosylated) | >85 % |
| Wild type afucosylated glyco-engineered humanized B-Ly1 (B-HH6-B-KV1) (non-afucosylated) | >85 % |
| afucosylated glyco-engineered humanized B-Ly1 (B-HH6-B-KV1 GE) | 45-50 % |

The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (variable region of the murine heavy chain (VH): SEQ ID NO:1; variable region of the murine light chain (VL): SEQ ID NO:2 (see Poppema, S. and Visser, L., Biotest Bulletin 3 (1987) 131-139) by chimerization with a human constant domain from IgG1 and following humanization (see WO 2005/044859 and WO 2007/031875). These "humanized B-Ly1 antibodies" are disclosed in detail in WO 2005/044859 and WO 2007/031875.

In one embodiment, the "humanized B-Ly1 antibody" has variable region of the heavy chain (VH) selected from group of of SEQ ID NO:3 to SEQ ID NO:19 (B-HH2 to B-HH9 and B-HL8 to B-HL17 of WO 2005/044859 and WO 2007/031875). In one specific embodiment, such variable domain is selected from the group consisting of SEQ ID NO:3, SEQ ID NO:4, SEQ ID NO:7, SEQ ID NO:9, SEQ ID NO:11, SEQ ID NO:13 and SEQ ID NO:15 (B-HH2, BHH-3, B-HH6, B-HH8, B-HL8, B-HL11 and B-HL13 of WO 2005/044859 and WO 2007/031875). In one specific embodiment, the "humanized B-Ly1 antibody" has variable region of the light chain (VL) of SEQ ID NO:20 (B-KV1 of WO 2005/044859 and WO 2007/031875). In one specific embodiment, the "humanized B-Ly1 antibody" has a variable region of the heavy chain (VH) of SEQ ID NO:7 (B-HH6 of WO 2005/044859 and WO 2007/031875) and a variable region of the light chain (VL) of SEQ ID NO:20 (B-KV1 of WO 2005/044859 and WO 2007/031875). Furthermore in one embodiment, the humanized B-Ly1 antibody is an IgG1 antibody. According to the invention such afucosylated humanized B-Ly1 antibodies are glycoengineered (GE) in the Fc region according to the procedures described in WO 2005/044859, WO 2004/065540, WO 2007/031875, Umana, P. et al., Nature Biotechnol. 17 (1999) 176-180 and WO 99/154342. In one embodiment, the afucosylated glyco-engineered humanized B-Ly1 is B-HH6-B-KV1 GE. In one embodiment, the anti-CD20 antibody is obinutuzumab (recommended INN, WHO Drug Information, Vol. 26, No. 4, 2012, p. 453). As used herein, obinutuzumab is synonymous for GA101. The tradename is GAZYVA or GAZYVARO. The WHO Drug Information document replaces all previous versions (e.g. Vol. 25, No. 1, 2011, p.75-76), and is formerly known as afutuzumab (recommended INN, WHO Drug Information, Vol. 23, No. 2, 2009, p. 176; Vol. 22, No. 2, 2008, p. 124).

The term "MDM2 inhibitor" according to the invention refers to agents that prevents activity of MDM2 with an IC50 of 0.001 µM to about 2 µM, in one embodiment with 0.002 µM to about 2 µM.

In another embodiment, the MDM2 inhibitors are small molecular weight compounds with a molecular weight (MW) of less than 1500 Daltons (Da).

According to some aspects of the disclosure, the MDM2 inhibitor is a compound selected from the compounds described in WO2011/098398. Methods of producing said MDM2 inhibitors are also disclosed in WO2011/098398. Said MDM2 inhibitor preferably is a compound according to formula I or according to formula Ia as disclosed herein (formula II or formula IIa of WO2011/098398). 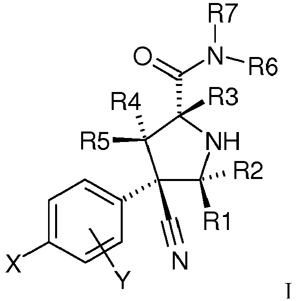 wherein
X is selected from the group consisting of H, F, Cl, Br, I, cyano, nitro, ethynyl, cyclopropyl, methyl, ethyl, isopropyl, vinyl and methoxy,
Y is one to four group(s) independently selected from the group consisting of H, F, Cl, Br, I, CN, OH, nitro, lower alkyl, cycloalkyl, lower alkoxy, lower alkenyl, cycloalkenyl, lower alkynyl, aryl, hetereoaryl, hetereocycle, COOR', OCOR', CONR'R", NR'COR", NR"SO₂R', SO₂NR'R" and NR'R" wherein
R' and R" are independently selected from H, lower alkyl, substituted lower alkyl, lower cycloalkyl, substituted lower cycloalkyl, lower alkenyl, substituted lower alkenyl, lower cycloalkenyl, substituted lower cycloalkenyl, aryl, substituted aryl, heteroaryl, substituted heteroaryl, hetereocycle, or substituted hetereocycle,
and wherein R^{'} and R^{"} may independently link to form a cyclic structure selected from substituted or unsubstituted cycloalkyl, substituted or unsubstituted cycloalkenyl, substituted or unsubstituted heteroaryl or substituted or unsubstituted heterocycle,
R₁ is selected from the group consisting of lower alkyl,
substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl, R₂ is hydrogen or lower alkyl,
R₃ is H or lower alkyl,
R₅ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl and substituted cycloalkenyl,
R₄ is hydrogen,
R₆ and R₇ are selected from the group consisting of (CH₂)ₙ-R', (CH₂)ₙ-NR'R", (CH₂)ₙ-NR'COR", (CH₂)ₙ-NR'SO₂R", (CH₂)ₙ-COOH, (CH₂)ₙ-COOR', (CH₂)ₙ-CONR'R", (CH₂)ₙ-OR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙ-OH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R', (CH₂)ₚ-(CH₂CH₂O)ₘ-(CH₂)ₙ-COR', (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R", -COR', -SOR' and SO₂R'
wherein R' and R" are as above,
m, n, and p are independently 0 to 6
and the pharmaceutically acceptable salts and esters thereof.

Especially preferred are compounds of formula I wherein
X is F, Cl or Br,
Y is one to two group(s) independently selected from the group consisting of H, F, Cl, Br, I, CN, OH, nitro, lower alkyl, cycloalkyl, lower alkoxy, lower alkenyl, lower cycloalkenyl and lower alkynyl,
R₁ is selected from the group consisting of lower alkyl,
substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl and substituted cycloalkenyl,
R₂ is hydrogen,
R₃ is H,
R₅ is selected from the group consisting of aryl, substituted aryl, heteroaryl and substituted heteroaryl,
R₄ is hydrogen,
R₆ and R₇ are selected from the group consisting of (CH₂)ₙ-R', (CH₂)ₙ-NR'R", (CH₂)ₙ-NR'COR", (CH₂)ₙ-NR'SO₂R", (CH₂)ₙ-COOH, (CH₂)ₙ-COOR', (CH₂)ₙ-CONR'R", (CH₂)ₙ-OR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙ-OH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R',
(CH₂)ₚ-(CH₂CH₂O)ₘ-(CH₂)ₙ-COR', (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R", -COR', -SOR' and SO₂R' wherein
R' and R" are independently selected from H, lower alkyl, substituted lower alkyl, lower cycloalkyl, substituted lower cycloalkyl, lower alkenyl, substituted lower alkenyl, lower cycloalkenyl, substituted lower cycloalkenyl, aryl, substituted aryl, heteroaryl, substituted heteroaryl, hetereocycle, or substituted hetereocycle,and wherein R^{'} and R^{"} may also independently link to form a cyclic structure selected from substituted or unsubstituted cycloalkyl, substituted or unsubstituted cycloalkenyl, substituted or unsubstituted heteroaryl or substituted or unsubstituted heterocycle,
m, n and p are independently 0 to 6
and the pharmaceutically acceptable salts and esters thereof.

Further preferred are compounds of formula I wherein:
X is F, Cl or Br,
Y is a mono-substituting group selected from H or F and
R₁ is selected from the group consisting of lower alkyl,
substituted lower alkyl, lower alkenyl, substituted lower alkenyl, heterocycle, substituted heterocycle, cycloalkyl, substituted cycloalkyl, cycloalkenyl and substituted cycloalkenyl.

Further preferred R₁ is a substituted lower alkyl selected from: 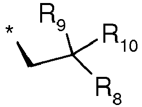
where R₈, R₉ are both methyl, or linked to form a cyclopropyl, cyclobutyl, cyclopentyl or acyclohexyl group,
R₁₀ is (CH₂)ₘ-R₁₁,
m is 0, 1 or 2,
R₁₁ is selected from hydrogen, hydroxyl, lower alkyl, lower alkoxy, aryl, substituted aryl. hetereoaryl, substituted heteroaryl, hetereocycle or substituted heterocycle,
R₂ is H,
R₃ is H,
R₅ is a substituted phenyl selected from: 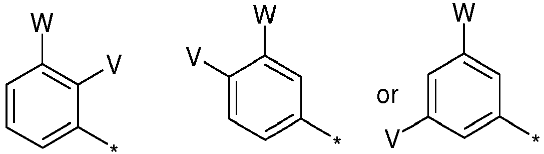
W is F, Cl or Br,
V is H or F,
R₄ is hydrogen,
one of R₆ and R₇ is hydrogen and the other is (CH₂)ₙ-R',
n is 0 or 1 and
R' is selected from aryl, substituted aryl, hetereoaryl, substituted heteroaryl, hetereocycle or substituted heterocycle.

In yet other aspects of the disclosure, the present invention is directed to the compounds of the formula **Ia** 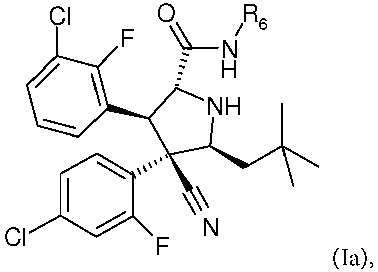 wherein R₆ and all variables and substituents mentioned in the definitions for R₆ have the meanings given for formula I above.

In still other aspects, there are provided the compounds of formula la, wherein R₆ is -(CH₂)ₙ-R', and
R' is Cyclohexyl, or
   a 5 to 10 membered, mono- or bicyclic aromatic hydrocarbon wherein 1 or 2 carbon atoms may be replaced by N, S or O, and wherein any of the aforementioned cyclohexyl or aromatic hydrocarbon can be substituted once or twice with a group independently selected from lower alkyl, lower-alkenyl, lower-alkynyl, dioxo-lower-alkylene (forming e.g. a benzodioxyl group), halogen, hydroxy, CN, CF₃, NH₂, N(H, lower-alkyl), N(lower-alkyl)₂, aminocarbonyl, carboxy, NO₂, lower-alkoxy, thio-lower-alkoxy, lower-alkylsufonyl, aminosulfonyl, lower-alkylcarbonyl, lower-alkylcarbonyloxy, lower-alkoxycarbonyl, lower-alkyl-carbonyl-NH, fluoro-lower-alkyl, fluoro-lower-alkoxy, lower-alkoxy-carbonyl-lower-alkoxy, carboxy-lower-alkoxy, carbamoyl-lower-alkoxy, hydroxy-lower-alkoxy, NH₂-lower-alkoxy, N(H, lower-alkyl)-lower-alkoxy, N(lower-alkyl)₂-lower-alkoxy, lower-alkyl-1-oxiranyl-lower-alkoxy-lower-alkyl, 2-oxo-pyrrolidin-1-yl, (1,1-dioxo)-2-isothiazolidine, 3-lower-alkyl sulfinyl, a substituted or unsubstituted heterocyclic ring, a substituted or unsubstituted aryl ring, a substituted or unsubstituted heteroaryl ring, trifluoro-lower-alkylsulfonylamino-aryl, lower-alkyl sulfonylaminocarbonyl, lower-alkyl sulfonylaminocarbonyl-aryl, hydroxycarbamoyl-phenyl, benzyloxy-lower-alkoxy, mono- or di-lower alkyl substituted amino-sulfonyl and lower-alkyl which can optionally be substituted with halogen, hydroxy, NH₂, N(H, lower-alkyl) or N(lower-alkyl)₂; and
n is 0 or 1.

In still other aspects there are provided the compounds of formula la, wherein
R6 is -(CH₂)ₙ-R', and
R' is phenyl, pyridinyl, pyrazinyl or pyrimidinyl which can be each unsubstituted or once or twice substituted with a substituent independently selected from halogen, CI-6 alkoxy, CI-6 alkyl, hydroxycarbonyl, carboxy, carboxy CI-6 alkoxy, oxo and CN; and
n is 0.

Especially preferred are the compounds of formula la, selected from
rac-4-({[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-methyl)-cyclohexanecarboxylic acid methyl ester,
rac-4-({[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-methyl)-cyclohexanecarboxylic acid,
rac-4-({[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-methyl)-2-methoxy-benzoic acid methyl ester,
rac-4-({[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-methyl)-2-methoxy-benzoic acid,
rac-4-({[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-methyl)-2-fluoro-benzoic acid methyl ester,
rac-4-({[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-methyl)-2-fluoro-benzoic acid,
chiral 5-({[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-methyl)-pyridine-2-carboxylic acid methyl ester,
chiral 5-({[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-methyl)-pyridine-2-carboxylic acid,
chiral 6-({[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-methyl)-nicotinic acid ethyl ester,
chiral 6-({[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-methyl)-nicotinic acid,
rac 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-2-ethoxy-benzoic acid,
chiral (2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-hydrazinocarbonyl-phenyl)-amide,
chiral [2-(4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-phenyl)-ethyl]-carbamic acid *tert*-butyl ester,
chiral (2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid [4-(2-amino-ethyl)-phenyl]-amide,
chiral 5-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-pyrazine-2-carboxylic acid,
chiral 4-(((2R,3S,4R,5S)-3-(3-chloro-2-fluorophenyl)-4-(4-chloro-2-fluorophenyl)-4-cyano-5-neopentylpyrrolidine-2-carboxamido)methyl)-2-methoxybenzoic acid,
chiral-4-({[(2S,3R,4S,5R)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-methyl)-2-methoxy-benzoic acid,
chiral methyl 3-(4-((2R,3S,4R,5S)-3-(3-chloro-2-fluorophenyl)-4-(4-chloro-2-fluorophenyl)-4-cyano-5-neopentylpyrrolidine-2-carboxamido)phenyl)propanoate,
chiral 3-(4-((2R,3S,4R,5S)-3-(3-chloro-2-fluorophenyl)-4-(4-chloro-2-fluorophenyl)-4-cyano-5-neopentylpyrrolidine-2-carboxamido)phenyl)propanoic acid,
chiral- 4-(((2S,3R,4S,5R)-3-(3-chloro-2-fluorophenyl)-4-(4-chloro-2-fluorophenyl)-4-cyano-5-neopentylpyrrolidine-2-carboxamido)methyl)-2-fluorobenzoic acid,
chiral 4-(((2R,3S,4R,5S)-3-(3-chloro-2-fluorophenyl)-4-(4-chloro-2-fluorophenyl)-4-cyano-5-neopentylpyrrolidine-2-carboxamido)methyl)-2-fluorobenzoic acid,
chiral (2R,3S,4R,5S)-3-(3-chloro-2-fluorophenyl)-4-(4-chloro-2-fluorophenyl)-4-cyano-N-(2-morpholinopyrimidin-5-yl)-5-neopentylpyrrolidine-2-carboxamide,
chiral (2R,3S,4R,5S)-3-(3-chloro-2-fluorophenyl)-4-(4-chloro-2-fluorophenyl)-4-cyano-5-neopentyl-N-(pyrimidin-5-yl)pyrrolidine-2-carboxamide,
chiral (2S,3R,4S,5R)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-dimethylaminomethyl-phenyl)-amide, (2R,3S,4R,5S)-4-(4-Chloro-2-fluorophenyl)-3-(3-chloro-2-fluoro-phenyl)-4-cyano-5-(2,2-dimethyl-propyl)-pyrrolidine-2-carboxylic acid (4-dimethylaminomethyl-phenyl)-amide,
chiral methyl 5-((2R,3S,4R,5S)-3-(3-chloro-2-fluorophenyl)-4-(4-chloro-2-fluorophenyl)-4-cyano-5-neopentylpyrrolidine-2-carboxamido)-1H-benzo[d] imidazole-2-carboxylate,
chiral-5-((2R,3S,4R,5S)-3-(3-chloro-2-fluorophenyl)-4-(4-chloro-2-fluorophenyl)-4-cyano-5-neopentylpyrrolidine-2-carboxamido)-1H-benzo[d]imidazole-2-carboxylic acid,
chiral-methyl 5-((2R,3S,4R,5S)-3-(3-chloro-2-fluorophenyl)-4-(4-chloro-2-fluorophenyl)-4-cyano-5-neopentylpyrrolidine-2-carboxamido)benzofuran-2-carboxylate,
chiral-5-((2R,3S,4R,5S)-3-(3-chloro-2-fluorophenyl)-4-(4-chloro-2-fluorophenyl)-4-cyano-5-neopentylpyrrolidine-2-carboxamido)benzofuran-2-carboxylic acid,
chiral- methyl 4-(4-((2R,3S,4R,5S)-3-(3-chloro-2-fluorophenyl)-4-(4-chloro-2-fluorophenyl)-4-cyano-5-neopentylpyrrolidine-2-carboxamido)phenyl)butanoate,
chiral- 4-(4-((2R,3S,4R,5S)-3-(3-chloro-2-fluorophenyl)-4-(4-chloro-2-fluorophenyl)-4-cyano-5-neopentylpyrrolidine-2-carboxamido)phenyl)butanoic acid,
chiral methyl 5-((2R,3S,4R,5S)-3-(3-chloro-2-fluorophenyl)-4-(4-chloro-2-fluorophenyl)-4-cyano-5-neopentylpyrrolidine-2-carboxamido)benzo[d]oxazole-2-carboxylate,
chiral- 5-((2R,3S,4R,5S)-3-(3-chloro-2-fluorophenyl)-4-(4-chloro-2-fluorophenyl)-4-cyano-5-neopentylpyrrolidine-2-carboxamido)benzo[d]oxazole-2-carboxylic acid,
chiral- (2R,3S,4R,5S)-N-(benzo[d]oxazol-5-yl)-3-(3-chloro-2-fluorophenyl)-4-(4-chloro-2-fluorophenyl)-4-cyano-5-neopentylpyrrolidine-2-carboxamide,
rac-(4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-benzyl)-carbamic acid tert-butyl ester,
rac-(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-aminomethylphenyl)-amide,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid [4-(methanesulfonylamino-methyl)-phenyl]-amide,
1-(4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-phenyl)-piperidine-4-carboxylic acid ethyl ester,
1-(4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-phenyl)-piperidine-4-carboxylic acid,
rac-(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-dimethylaminomethyl-phenyl)-amide,
rac-5-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-2-pyrrolidin-1-yl-benzoic acid,
rac-4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-4-methyl-piperidine-1-carboxylic acid tert-butyl ester,
rac- (2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-methyl-piperidin-4-yl)-amide,
rac-(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (1-methanesulfonyl-4-methyl-piperidin-4-yl)-amide,
methyl 1-(4-((2R,3S,4R,5S)-3-(3-chloro-2-fluorophenyl)-4-(4-chloro-2-fluorophenyl)-4-cyano-5-neopentylpyrrolidine-2-carboxamido)phenyl)pyrrolidine-2-carboxylate,
1-(4-((2R,3S,4R,5S)-3-(3-chloro-2-fluorophenyl)-4-(4-chloro-2-fluorophenyl)-4-cyano-5-neopentylpyrrolidine-2-carboxamido)phenyl)pyrrolidine-2-carboxylic acid,
chiral 5-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-1H-pyrrole-2-carboxylic acid,
chiral 5-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-1H-pyrrole-2-carboxylic acid ethyl ester,
chiral (*R*)-2-(4-{[(2*R*,3S,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-phenyl)-butyric acid,
chiral (*S*)-2-(4-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-phenyl)-butyric acid,
chiral (*S*)-2-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-3-thiazol-4-yl-propionic acid methyl ester,
chiral (*S*)-2-{[(2*S*,3*R*,4*S*,5*R*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-3-thiazol-4-yl-propionic acid methyl ester,
chiral (*S*)-2-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-3-thiazol-4-yl-propionic acid,
chiral (*S*)-2-{[(2*S*,3*R*,4*S*,5*R*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-3-thiazol-4-yl-propionic acid,
chiral 4-{[(2R,3S,4R,5S)-3-(3-chloro-2-fluoro-phenyl)-4-(4-chloro-2-fluorophenyl)-4-cyano-5-(2,2-dimethyl-propyl)-pyrrolidine-2-carbonyl]-amino}-1H-indole-2-carboxylic acid,
rac (2R,3S,4R,5S)-4-(4-chloro-2-fluoro-phenyl)-3-(3-chloro-2-fluoro-phenyl)-4-cyano-5-(2,2-dimethyl-propyl)-pyrrolidine-2-carboxylic acid (5-iodo-pyridin-2-yl)-amide,
2-chloro-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
6-{[(2R,3S,4R,5S)-4-(4-chloro-2-fluoro-phenyl)-3-(3-chloro-2-fluoro-phenyl)-4-cyano-5-(2,2-dimethyl-propyl)-pyrrolidine-2-carbonyl]-amino}-nicotinic acid,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid pyridin-2-ylamide,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid pyridin-4-ylamide,
5-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-pyridine-2-carboxylic acid,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid pyridin-3-ylamide,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-iodo-3,5dimethyl-phenyl)-amide,
4-{[(2R,3S,4R,5S)-4-(4-Chloro-2-fluoro-phenyl)-3-(3-chloro-2 fluoro-phenyl)-4-cyano-5-(2,2-dimethyl-propyl)-pyrrolidine-2-carbonyl]-amino}-2-fluoro-benzoic acid tert-butyl ester,
4-{[(2R,3S,4R,5S)-4-(4-Chloro-2-fluoro-phenyl)-3-(3-chloro-2 fluoro-phenyl)-4-cyano-5-(2,2-dimethyl-propyl)-pyrrolidine-2-carbonyl]-amino}-2-fluoro-benzoic acid,
4-{[(2R,3S,4R,5S)-4-(4-Chloro-2-fluoro-phenyl)-3-(3-chloro-2 fluoro-phenyl)-4-cyano-5-(2,2-dimethyl-propyl)-pyrrolidine-2-carbonyl]-amino}-3-fluoro-benzoic acid,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-2-trifluoromethyl-benzoic acid,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-iodo-2-trifluoromethoxy-phenyl)-amide,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-trifluoromethoxy-benzoic acid methyl ester,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-trifluoromethoxy-benzoic acid,
6-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-nicotinic acid,
6-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-nicotinic acid methyl ester,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (6-iodo-pyridin-3-yl)-amide,
5-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-pyridine-2-carboxylic acid methyl ester,
4-{[(2R,3S,4R,5S)-4-(4-chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid methyl ester,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (6-carbamoyl-naphthalen-2-yl)-amide,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-carbamoyl-3-chloro-phenyl)-amide,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-carbamoyl-3-trifluoromethyl-phenyl)-amide,
5-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-pyridine-2-carboxylic acid,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-fluoro-benzoic acid methyl ester,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-chloro-benzoic acid methyl ester,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-chloro-benzoic acid,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-carbamoyl-2-fluoro-phenyl)-amide,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-carbamoyl-2-methoxy-phenyl)-amide,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-2,5-difluoro-benzoic acid methyl ester,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-2,5-difluoro-benzoic acid,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (3,5-difluoro-4-iodo-phenyl)-amide,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-2,6-difluoro-benzoic acid,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-hydroxy-benzoic acid,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-carbamoyl-3-methoxy-phenyl)-amide,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-carbamoyl-3-trifluoromethoxy-phenyl)-amide,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-carbamoyl-3-fluoro-phenyl)-amide,
(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carboxylic acid (4-carbamoyl-2-chloro-phenyl)-amide,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-2-fluoro-5-methoxy-benzoic acid methyl ester,
4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-2-fluoro-5-methoxy-benzoic acid,
2-(4-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-phenyl)-4-methyl-pentanoic acid,
chiral 2-(4-{[(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-phenyl)-2-methyl-propionic acid methyl ester,
chiral 2-(4-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-phenyl)-2-methyl-propionic acid,
chiral (2*S*,3*R*,4*S*,5*R*)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(1-methyl-1-methylcarbamoyl-ethyl)-phenyl]-amide,
chiral (2*S*,3*R*,4*S*,5*R*)-3-(3-chloro-2-fluoro-phenyl)-4-(4-chloro-2-fluoro-phenyl)-4-cyano-5-(2,2-dimethyl-propyl)-pyrrolidine-2-carboxylic acid {4-[1-(3-hydroxy-propylcarbamoyl)-1-methyl-ethyl]-phenyl}amide, and
chiral (2*S*,3*R*,4*S*,5*R*)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(1-carbamoyl-1-methyl-ethyl)-phenyl]-amide.

In the specification where indicated the various groups may be substituted by 1-5 or, preferably, 1-3 substituents independently selected from the group consisting of lower alkyl, lower-alkenyl, lower-alkynyl, dioxo-lower-alkylene (forming e.g. a benzodioxyl group), halogen, hydroxy, CN, CF₃, NH₂, N(H, lower-alkyl), N(lower-alkyl)₂, aminocarbonyl, carboxy, NO₂, lower-alkoxy, thio-lower-alkoxy, lower-alkylsufonyl, aminosulfonyl, lower-alkylcarbonyl, lower-alkylcarbonyloxy, lower-alkoxycarbonyl, lower-alkyl-carbonyl-NH, fluoro-lower-alkyl, fluoro-lower-alkoxy, lower-alkoxy-carbonyl-lower-alkoxy, carboxy-lower-alkoxy, carbamoyl-lower-alkoxy, hydroxy-lower-alkoxy, NH₂-lower-alkoxy, N(H, lower-alkyl)-lower-alkoxy, N(lower-alkyl)₂-lower-alkoxy, lower-alkyl-1-oxiranyl-lower-alkoxy-lower-alkyl, 2-oxo-pyrrolidin-1-yl, (1,1-dioxo)-2-isothiazolidine, 3-lower-alkyl sulfinyl, a substituted or unsubstituted heterocyclic ring, a substituted or unsubstituted aryl ring, a substituted or unsubstituted heteroaryl ring, trifluoro-lower-alkylsulfonylamino-aryl, lower-alkyl sulfonylaminocarbonyl, lower-alkyl sulfonylaminocarbonyl-aryl, hydroxycarbamoyl-phenyl, benzyloxy-lower-alkoxy, mono- or di-lower alkyl substituted amino-sulfonyl and lower-alkyl which can optionally be substituted with halogen, hydroxy, NH₂, N(H, lower-alkyl) or N(lower-alkyl)_{2.}. Preferred substituents for the cycloalkyl, cycloalkenyl, aryl, heteroaryl and heterocycle rings are halogen, lower alkoxy, lower alkyl, hydroxycarbonyl, carboxy, carboxy lower alkoxy, oxo and CN. Preferred substituents for alkyl are alkoxy and N(lower alkyl)₂.

The term "alkyl" refers to straight- or branched-chain saturated hydrocarbon groups having from 1 to about 20 carbon atoms, including groups having from 1 to about 7 carbon atoms. In certain aspects, alkyl substituents may be lower alkyl substituents. The term "lower alkyl" refers to alkyl groups having from 1 to 6 carbon atoms, and in certain aspects from 1 to 4 carbon atoms. Examples of alkyl groups include, but are not limited to, methyl, ethyl, n-propyl, i-propyl, n-butyl, s-butyl, t-butyl, n-pentyl, and s-pentyl.

As used herein, "cycloalkyl" is intended to refer to any stable monocyclic or polycyclic system which consists of carbon atoms only, any ring of which being saturated, and the term "cycloalkenyl" is intended to refer to any stable monocyclic or polycyclic system which consists of carbon atoms only, with at least one ring thereof being partially unsaturated. Examples of cycloalkyls include, but are not limited to, cyclopropyl, cyclobutyl, cyclopentyl, cyclohexyl, cycloheptyl, adamantyl, cyclooctyl, bicycloalkyls, including bicyclooctanes such as [2.2.2]bicyclooctane or [3.3.0]bicyclooctane, bicyclononanes such as [4.3.0]bicyclononane, and bicyclodecanes such as [4.4.0]bicyclodecane (decalin), or spiro compounds. Examples of cycloalkenyls include, but are not limited to, cyclopentenyl or cyclohexenyl.

The term "alkenyl" as used herein means an unsaturated straight-chain or branched aliphatic hydrocarbon group containing one double bond and having 2 to 6, preferably 2 to 4 carbon atoms. Examples of such "alkenyl group" are vinyl ethenyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl and 5-hexenyl.

The term "alkynyl" as used herein means an unsaturated straight-chain or branched aliphatic hydrocarbon group containing one triple bond and having 2 to 6, preferably 2 to 4 carbon atoms. Examples of such "alkynyl group" are ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl.

The term "halogen" as used in the definitions means fluorine, chlorine, bromine, or iodine, preferably fluorine and chlorine.

"Aryl" means a monovalent, monocyclic or bicyclic, aromatic carbocyclic hydrocarbon radical, preferably a 6-10 member aromatic ring system. Preferred aryl groups include, but are not limited to, phenyl, naphthyl, tolyl, and xylyl. Where the aryl group is bicyclic a preferred group is 1,3-dioxo-2,3-dihydro-1H-isoindol-5-yl group.

"Heteroaryl" means an aromatic heterocyclic ring system containing up to two rings. Preferred heteroaryl groups include, but are not limited to, thienyl, furyl, indolyl, pyrrolyl, pyridinyl, pyrazinyl, oxazolyl, thiaxolyl, quinolinyl, pyrimidinyl, imidazole substituted or unsubstituted triazolyl and substituted or unsubstituted tetrazolyl.

In the case of aryl or heteroaryl which are bicyclic it should be understood that one ring may be aryl while the other is heteroaryl and both being substituted or unsubstituted.

"Heterocycle" or "heterocyclic ring"means a substituted or unsubstituted 5 to 8 membered, mono- or bicyclic, non-aromatic hydrocarbon, wherein 1 to 3 carbon atoms are replaced by a hetero atom selected from nitrogen, oxygen or sulfur atom. Examples include pyrrolidin-2-yl; pyrrolidin-3-yl; piperidinyl; morpholin-4-yl and the like which in turn can be substituted. "Hetero atom" means an atom selected from N, O and S.

"Alkoxy, alkoxyl or lower alkoxy" refers to any of the above lower alkyl groups attached to an oxygen atom. Typical lower alkoxy groups include methoxy, ethoxy, isopropoxy or propoxy, butyloxy and the like. Further included within the meaning of alkoxy are multiple alkoxy side chains, *e.g.* ethoxy ethoxy, methoxy ethoxy, methoxy ethoxy ethoxy and the like and substituted alkoxy side chains, e.g., dimethylamino ethoxy, diethylamino ethoxy, dimethoxy-phosphoryl methoxy and the like.

"Pharmaceutically acceptable," such as pharmaceutically acceptable carrier, excipient, etc., means pharmacologically acceptable and substantially non-toxic to the subject to which the particular compound is administered.

"Pharmaceutically acceptable salt" refers to conventional acid-addition salts or base-addition salts that retain the biological effectiveness and properties of the compounds of the present invention and are formed from suitable non-toxic organic or inorganic acids or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, trifluoro acetic acid and the like. Sample base-addition salts include those derived from ammonium, potassium, sodium and, quaternary ammonium hydroxides, such as for example, tetramethylammonium hydroxide. Chemical modification of a pharmaceutical compound (i.e. drug) into a salt is a technique well known to pharmaceutical chemists to obtain improved physical and chemical stability, hygroscopicity, flowability and solubility of compounds. *See, e.g.,* Ansel et al., Pharmaceutical Dosage Forms and Drug Delivery Systems (6th Ed. 1995) at pp. 196 and 1456- 1457.

The compounds of formula **I** or **Ia** as well as their salts that have at least one asymmetric carbon atom may be present as racemic mixtures or different stereoisomers. The various isomers can be isolated by known separation methods, e.g., chromatography.

Compounds disclosed herein and covered by formula **I** or **Ia** above may exhibit tautomerism or structural isomerism. It is intended that these formulas encompass any tautomeric or structural isomeric form of these compounds, or mixtures of such forms, and is not limited to any one tautomeric or structural isomeric form depicted in the formulas above.

Most preferably, the MDM2 inhibitor is 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino }-3-methoxy-benzoic acid as in the formula below or a salt, ester or polymorph thereof. Example 448 of WO2011/098398 describes a method for preparation of said compound. Said compound is also named MDM2(4) or RG7388 or RO5503781 as used herein as synonymous terms.

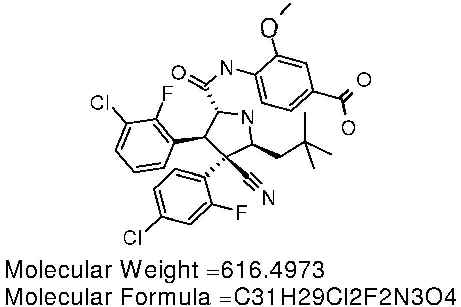

Preparation of the MDM2 inhibitor is carried out as disclosed in WO2011/098398.

"Salt" refers to salts of the compounds as a pharmaceutically acceptable salt. Such salts can be exemplified by the salts with alkali metals (potassium, sodium, and the like), salts with alkaline-earth metals (calcium, magnesium, and the like), the ammonium salt, salts with pharmaceutically acceptable organic amines (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine, and the like), and acid addition salts (inorganic acid salts (the hydrochloride, hydrobromide, hydroiodide, sulfate, phosphate, nitrate, and the like) and organic acid salts (the acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate, and the like)).

"IC50" refers to the concentration of a particular compound required to inhibit 50% of a specific measured activity.

The oligosaccharide component can significantly affect properties relevant to the efficacy of a therapeutic glycoprotein, including physical stability, resistance to protease attack, interactions with the immune system, pharmacokinetics, and specific biological activity. Such properties may depend not only on the presence or absence, but also on the specific structures, of oligosaccharides. Some generalizations between oligosaccharide structure and glycoprotein function can be made. For example, certain oligosaccharide structures mediate rapid clearance of the glycoprotein from the bloodstream through interactions with specific carbohydrate binding proteins, while others can be bound by antibodies and trigger undesired immune reactions (Jenkins, N., et al., Nature Biotechnol. 14 (1996) 975-981).

Mammalian cells are the excellent hosts for production of therapeutic glycoproteins, due to their capability to glycosylate proteins in the most compatible form for human application (Cumming, D.A., et al., Glycobiology 1 (1991) 115-130; Jenkins, N., et al., Nature Biotechnol. 14 (1996) 975-981). Bacteria very rarely glycosylate proteins, and like other types of common hosts, such as yeasts, filamentous fungi, insect and plant cells, yield glycosylation patterns associated with rapid clearance from the blood stream, undesirable immune interactions, and in some specific cases, reduced biological activity. Among mammalian cells, Chinese hamster ovary (CHO) cells have been most commonly used during the last two decades. In addition to giving suitable glycosylation patterns, these cells allow consistent generation of genetically stable, highly productive clonal cell lines. They can be cultured to high densities in simple bioreactors using serum free media, and permit the development of safe and reproducible bioprocesses. Other commonly used animal cells include baby hamster kidney (BHK) cells, NSO- and SP2/0-mouse myeloma cells. More recently, production from transgenic animals has also been tested (Jenkins, N., et al., Nature Biotechnol. 14 (1996) 975-981).

All antibodies contain carbohydrate structures at conserved positions in the heavy chain constant regions, with each isotype possessing a distinct array of N-linked carbohydrate structures, which variably affect protein assembly, secretion or functional activity (Wright, A., and Morrison, S.L., Trends Biotech. 15 (1997) 26-32). The structure of the attached N-linked carbohydrate varies considerably, depending on the degree of processing, and can include high-mannose, multiply-branched as well as biantennary complex oligosaccharides (Wright, A., and Morrison, S.L., Trends Biotech. 15 (1997) 26-32). Typically, there is heterogeneous processing of the core oligosaccharide structures attached at a particular glycosylation site such that even monoclonal antibodies exist as multiple glycoforms. Likewise, it has been shown that major differences in antibody glycosylation occur between cell lines, and even minor differences are seen for a given cell line grown under different culture conditions (Lifely, M.R., et al., Glycobiology 5 (1995) 813-822).

One way to obtain large increases in potency, while maintaining a simple production process and potentially avoiding significant, undesirable side effects, is to enhance the natural, cell-mediated effector functions of monoclonal antibodies by engineering their oligosaccharide component as described in Umana, P. et al., Nature Biotechnol. 17 (1999) 176-180 and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M.R., et al., Glycobiology 5 (1995) 813-822; Jefferis, R., et al., Immunol. Rev. 163 (1998) 59-76; Wright, A. and Morrison, S.L., Trends Biotechnol. 15 (1997) 26-32).

It was previously shown that overexpression in Chinese hamster ovary (CHO) cells of β(1,4)-N-acetylglucosaminyltransferase III ("GnTIII7y), a glycosyltransferase catalyzing the formation of bisected oligosaccharides, significantly increases the in vitro ADCC activity of an antineuroblastoma chimeric monoclonal antibody (chCE7) produced by the engineered CHO cells (see Umana, P. et al., Nature Biotechnol. 17 (1999) 176-180; and WO 99/154342). The antibody chCE7 belongs to a large class of unconjugated monoclonal antibodies which have high tumor affinity and specificity, but have too little potency to be clinically useful when produced in standard industrial cell lines lacking the GnTIII enzyme (Umana, P., et al., Nature Biotechnol. 17 (1999) 176-180). That study was the first to show that large increases of ADCC activity could be obtained by engineering the antibody producing cells to express GnTIII, which also led to an increase in the proportion of constant region (Fc)-associated, bisected oligosaccharides, including bisected, non-fucosylated oligosaccharides, above the levels found in naturally-occurring antibodies.

The term "cancer" as used herein includes lymphomas, lymphocytic leukemias, lung cancer, non small cell lung (NSCL) cancer, bronchioloalveolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymomas, medulloblastomas, meningiomas, squamous cell carcinomas, pituitary adenoma, including refractory versions of any of the above cancers, or a combination of one or more of the above cancers. In one embodiment, the term cancer refers to a CD20 expressing cancer.

The term "expression of the CD20" antigen is intended to indicate an significant level of expression of the CD20 antigen in a cell, preferably on the cell surface of a T- or B- cell, more preferably a B-cell, from a tumor or cancer, respectively, preferably a non-solid tumor. Patients having a "CD20 expressing cancer" can be determined by standard assays known in the art. For example CD20 antigen expression can be measured using immunohistochemical (IHC) detection, FACS or via PCR-based detection of the corresponding mRNA.

The term "CD20 expressing cancer" as used herein refers to all cancers in which the cancer cells show an expression of the CD20 antigen. Preferably CD20 expressing cancer as used herein refers to lymphomas (preferably B-Cell Non-Hodgkin's lymphomas (NHL)) and lymphocytic leukemias. Such lymphomas and lymphocytic leukemias include e.g. a) follicular lymphomas, b) Small Non-Cleaved Cell Lymphomas/ Burkitt's lymphoma (including endemic Burkitt's lymphoma, sporadic Burkitt's lymphoma and Non-Burkitt's lymphoma), c) marginal zone lymphomas (including extranodal marginal zone B cell lymphoma (Mucosa-associated lymphatic tissue lymphomas, MALT), nodal marginal zone B cell lymphoma and splenic marginal zone lymphoma), d) Mantle cell lymphoma (MCL), e) Large Cell Lymphoma (including B-cell diffuse large cell lymphoma (DLCL), Diffuse Mixed Cell Lymphoma, Immunoblastic Lymphoma, Primary Mediastinal B-Cell Lymphoma, Angiocentric Lymphoma-Pulmonary B-Cell Lymphoma), f) hairy cell leukemia, g) lymphocytic lymphoma, waldenstrom's macroglobulinemia, h) acute lymphocytic leukemia (ALL), chronic lymphocytic leukemia (CLL)/ small lymphocytic lymphoma (SLL), B-cell prolymphocytic leukemia, i) plasma cell neoplasms, plasma cell myeloma, multiple myeloma, plasmacytoma, j) Hodgkin's disease.

In one embodiment, the CD20 expressing cancer is a B-Cell Non-Hodgkin's lymphomas (NHL). In another embodiment, the CD20 expressing cancer is a Mantle cell lymphoma (MCL), acute lymphocytic leukemia (ALL), chronic lymphocytic leukemia (CLL), B-cell diffuse large cell lymphoma (DLCL), Burkitt's lymphoma, hairy cell leukemia, follicular lymphoma, multiple myeloma, marginal zone lymphoma, post transplant lymphoproliferative disorder (PTLD), HIV associated lymphoma, waldenstrom's macroglobulinemia, or primary CNS lymphoma.

The term "a method of treating", "a method of treatment" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The terms "combination", "co-administration" or "co-administering " refer to the administration of said type I anti-CD20 antibody or said afucosylated anti-CD20, and said Bcl-2 inhibitor and said MDM2 inhibitor as two separate formulations (or as one single formulation). The co-administration can be simultaneous or sequential in either order, wherein preferably there is a time period while both (or all) active agents simultaneously exert their biological activities. Said type I anti-CD20 antibody or said anti-CD20 afucosylated antibody and said Bcl-2 inhibitor and said MDM2 inhibitor are co-administered either simultaneously or sequentially (e.g. intravenous (i.v.) through a continuous infusion (one for the anti-CD20 antibody and eventually one for said Bcl-2 inhibitor and said MDM2 inhibitor; or e.g. the anti-CD20 antibody is administered intravenous (i.v.) through a continuous infusion and said Bcl-2 inhibitor and said MDM2 inhibitor are administered orally). When both therapeutic agents are co-administered sequentially, the dose is administered either on the same day in two separate administrations, or one of the agents is administered on day 1 and the second is co-administered on day 2 to day 7, preferably on day 2 to 4. Thus the term "sequentially" means within 7 days after the dose of the first component (anti-CD20 antibody or said Bcl-2 inhibitor and said MDM2 inhibitor), preferably within 4 days after the dose of the first component; and the term "simultaneously" means at the same time. The terms "co-administration" with respect to the maintenance doses of said type I anti-CD20 antibody or said afucosylated anti-CD20 antibody and said Bcl-2 inhibitor and said MDM2 inhibitor mean that the maintenance doses can be either co-administered simultaneously, if the treatment cycle is appropriate for both drugs, e.g. every week. Or said Bcl-2 inhibitor and said MDM2 inhibitor are administered e.g. every first to third day and said afucosylated antibody is administered every week. Or the maintenance doses are co-administered sequentially, either within one or within several days.

It is self-evident that the antibodies are administered to the patient in a "therapeutically effective amount" (or simply "effective amount") which is the amount of the respective compound or combination that will elicit the biological or medical response of a tissue, system, animal or human that is being sought by the researcher, veterinarian, medical doctor or other clinician.

The amount of co-administration of said type I anti-CD20 antibody or said anti-CD20 afucosylated antibody and said Bcl-2 inhibitor and said MDM2 inhibitor inhibitor and the timing of co-administration will depend on the type (species, gender, age, weight, etc.) and condition of the patient being treated and the severity of the disease or condition being treated. Said type I anti-CD20 antibody or said afucosylated anti-CD20 antibody and said Bcl-2 inhibitor and said MDM2 inhibitor are suitably co-administered to the patient at one time or over a series of treatments e.g. on the same day or on the day after.

If the administration is intravenous the initial infusion time for said type I anti-CD20 antibody, said afucosylated anti-CD20 antibody or said Bcl-2 inhibitor and said MDM2 inhibitor inhibitor may be longer than subsequent infusion times, for instance approximately 90 minutes for the initial infusion, and approximately 30 minutes for subsequent infusions (if the initial infusion is well tolerated). Depending on the type and severity of the disease, about 0.1 mg /kg to 50 mg/kg (e.g. 0.1-20 mg/kg) of said type I anti-CD20 antibody or afucosylated anti-CD20 antibody; and 1 µg /kg to 50 mg/kg (e.g. 0.1-20 mg/kg) of said Bcl-2 inhibitor and said MDM2 inhibitor inhibitor is an initial candidate dosage for co-administration of both drugs to the patient. In one embodiment the preferred dosage of said afucosylated anti-CD20 antibody (preferably the afucosylated humanized B-Ly1 antibody, more preferably obinutuzumab) will be in the range from about 0.05mg/kg to about 30mg/kg. Thus, one or more doses of about 0.5mg/kg, 2.0mg/kg, 4.0mg/kg, 10mg/kg or 30mg/kg (or any combination thereof) may be co-administered to the patient.

In one aspect the preferred dosage of said Bcl-2 inhibitor (preferably 4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)-N-({4-[(tetrahydro-2H-pyran-4-ylmethyl)amino]-3-[(trifluoromethyl)sulfonyl]phenyl}sulfonyl)benzamide or a salt or polymorph thereof) will be in the range from about 0.05mg/kg to about 30mg/kg. Thus, one or more doses of about 0.5mg/kg, 2.0mg/kg, 4.0mg/kg, 10mg/kg or 30mg/kg (or any combination thereof) may be co-administered to the patient.

In one aspect the preferred dosage of said MDM2 inhibitor (preferably 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid or a salt or polymorph thereof) will be in the range from about 0.05mg/kg to about 30mg/kg. Thus, one or more doses of about 0.5mg/kg, 2.0mg/kg, 4.0mg/kg, 10mg/kg or 30mg/kg (or any combination thereof) may be co-administered to the patient.

Depending on the type (species, gender, age, weight, etc.) and condition of the patient and on the type I anti-CD20 antibody, preferably rituximub or type of afucosylated anti-CD20 antibody, preferably the afucosylated humanized B-Ly1 antibody, more preferably obinutuzumab, the dosage and the administration schedule of said type I anti-CD20 antibody or said afucosylated anti-CD20 antibody can differ from said Bcl-2 inhibitor and said MDM2 inhibitor. E.g. the type I anti-CD20 antibody or said afucosylated anti-CD20 antibody may be administered e.g. every one to three weeks and said Bcl-2 inhibitor and said MDM2 inhibitor may be administered daily or every 2 to 10 days. An initial higher loading dose, followed by one or more lower doses may also be administered.

The dosage of type I anti-CD20 antibody (preferably rituximab) may be in the range from about 100mg/m² to about 1000mg/m² on day 1, 8, 15, 22, 29, 36, 43, 50, 57 of a 8-weeks-dosage-cycle.

In one embodiment the preferred dosage of said afucosylated anti-CD20 antibody (preferably the afucosylated humanized B-Ly1 antibody, more preferably obinutuzumab) will be 800 to 1600 mg (in on embodiment 800 to 1200 mg) on day 1, 8, 15 of a 3- to 6-weeks-dosage-cycle and then in a dosage of 400 to 1200 (in one embodiment 800 to 1200 mg on day 1 of up to nine 3- to 4-weeks-dosage-cycles.

In one aspect the dose for said Bcl-2 inhibitor, wherein the Bcl-2 inhibitor is selected from the group consisting of the Bcl-2 inhibitors as described above and is preferably 4-(4-{[2-(4-chlorophenyl)-4,4-dimethylcyclohex-1-en-1-yl]methyl} piperazin-1-yl)-2-(1H-pyrrolo[2,3-b]pyridin-5-yloxy)-N-({4-[(tetrahydro-2H-pyran-4-ylmethyl)amino]-3-[(trifluoromethyl)sulfonyl]phenyl}sulfonyl)benzamide, is as follows. Said dose according to the invention is 10 mg/kg to 70 mg/kg, preferably 20 mg/kg to 55 mg/kg, once daily or every other day as oral administration.

In one aspect the dose for said MDM2 inhibitor, wherein a MDM2 inhibitor is selected from the group consisting of the MDM2 inhibitors as described above and is preferably 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid, is as follows. Said dose according to the invention is 10 mg/kg to 70 mg/kg, preferably 20 mg/kg to 55 mg/kg, once daily or every other day as oral administration.

The recommended dose may vary whether there is a further co-administration of chemotherapeutic agent and based on the type of chemotherapeutic agent.

In a embodiment, this invention is useful for preventing or reducing metastasis or further dissemination in such a patient suffering from cancer, preferably CD20 expressing cancer. This invention is useful for increasing the duration of survival of such a patient, increasing the progression free survival of such a patient, increasing the duration of response, resulting in a statistically significant and clinically meaningful improvement of the treated patient as measured by the duration of survival, progression free survival, response rate or duration of response. In a preferred embodiment, this invention is useful for increasing the response rate in a group of patients.

In the context of this disclosure, additional other cytotoxic, chemotherapeutic or anti-cancer agents, or compounds or ionizing radiation that enhance the effects of such agents (e.g. cytokines) may be used in the type I anti-CD20 antibody or the afucosylated anti-CD20 antibody and said Bcl-2 inhibitor and said MDM2 inhibitor combination treatment of cancer. Such molecules are suitably present in combination in amounts that are effective for the purpose intended. In one embodiment, said type I anti-CD20 antibody, preferably rituximab or said afucosylated anti-CD20 antibody, preferably the afucosylated humanized B-Ly1 antibody, more preferably obinutuzumab, and said Bcl-2 inhibitor and said MDM2 inhibitor combination treatment is used without such additional cytotoxic, chemotherapeutic or anti-cancer agents, or compounds that enhance the effects of such additional agents.

Such additional agents include, for example: alkylating agents or agents with an alkylating action, such as cyclophosphamide (CTX; e.g. cytoxan®), chlorambucil (CHL; e.g. leukeran®), cisplatin (CisP; e.g. platinol®) busulfan (e.g. myleran®), melphalan, carmustine (BCNU), streptozotocin, triethylenemelamine (TEM), mitomycin C, and the like; anti-metabolites, such as methotrexate (MTX), etoposide (VP16; e.g. vepesid®), 6-mercaptopurine (6MP), 6-thiocguanine (6TG), cytarabine (Ara-C), 5-fluorouracil (5-FU), capecitabine (e.g. Xeloda®), dacarbazine (DTIC), and the like; antibiotics, such as actinomycin D, doxorubicin (DXR; e.g. adriamycin®), daunorubicin (daunomycin), bleomycin, mithramycin and the like; alkaloids, such as vinca alkaloids such as vincristine (VCR), vinblastine, and the like; and other antitumor agents, such as paclitaxel (e.g. taxol®) and paclitaxel derivatives, the cytostatic agents, glucocorticoids such as dexamethasone (DEX; e.g. decadron®) and corticosteroids such as prednisone, nucleoside enzyme inhibitors such as hydroxyurea, amino acid depleting enzymes such as asparaginase, leucovorin and other folic acid derivatives, and similar, diverse antitumor agents. The following agents may also be used as additional agents: arnifostine (e.g. ethyol®), dactinomycin, mechlorethamine (nitrogen mustard), streptozocin, cyclophosphamide, lomustine (CCNU), doxorubicin lipo (e.g. doxil®), gemcitabine (e.g. gemzar®), daunorubicin lipo (e.g. daunoxome®), procarbazine, mitomycin, docetaxel (e.g. taxotere®), aldesleukin, carboplatin, oxaliplatin, cladribine, camptothecin, CPT 11 (irinotecan), 10-hydroxy 7-ethyl-camptothecin (SN38), floxuridine, fludarabine, ifosfamide, idarubicin, mesna, interferon beta, interferon alpha, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, chlorambucil. In one embodiment, the afucosylated anti-CD20 antibody and said Bcl-2 inhibitor and said MDM2 inhibitor combination treatment is used without such additional agents.

The use of the cytotoxic and anticancer agents described above as well as antiproliferative target-specific anticancer drugs like protein kinase inhibitors in chemotherapeutic regimens is generally well characterized in the cancer therapy arts, and their use herein falls under the same considerations for monitoring tolerance and effectiveness and for controlling administration routes and dosages, with some adjustments. For example, the actual dosages of the cytotoxic agents may vary depending upon the patient's cultured cell response determined by using histoculture methods. Generally, the dosage will be reduced compared to the amount used in the absence of additional other agents.

Typical dosages of an effective cytotoxic agent can be in the ranges recommended by the manufacturer, and where indicated by in vitro responses or responses in animal models, can be reduced by up to about one order of magnitude concentration or amount. Thus, the actual dosage will depend upon the judgment of the physician, the condition of the patient, and the effectiveness of the therapeutic method based on the in vitro responsiveness of the primary cultured malignant cells or histocultured tissue sample, or the responses observed in the appropriate animal models.

In the context of this disclosure, an effective amount of ionizing radiation may be carried out and/or a radiopharmaceutical may be used in addition to the type I anti-CD20 antibody or the afucosylated anti-CD20 antibody and said Bcl-2 inhibitor and said MDM2 inhibitor combination treatment of CD20 expressing cancer. The source of radiation can be either external or internal to the patient being treated. When the source is external to the patient, the therapy is known as external beam radiation therapy (EBRT). When the source of radiation is internal to the patient, the treatment is called brachytherapy (BT). Radioactive atoms for use in the context of this invention can be selected from the group including, but not limited to, radium, yttrium-90, cesium-137, iridium-192, americium-241, gold-198, cobalt-57, copper-67, technetium-99, iodine-123, iodine-131, and indium-111. Is also possible to label the antibody with such radioactive isotopes. In one embodiment, the type I anti-CD20 antibody or the afucosylated anti-CD20 antibody said Bcl-2 inhibitor and said MDM2 inhibitor combination treatment is used without such ionizing radiation.

Radiation therapy is a standard treatment for controlling unresectable or inoperable tumors and/or tumor metastases. Improved results have been seen when radiation therapy has been combined with chemotherapy. Radiation therapy is based on the principle that high-dose radiation delivered to a target area will result in the death of reproductive cells in both tumor and normal tissues. The radiation dosage regimen is generally defined in terms of radiation absorbed dose (Gy), time and fractionation, and must be carefully defined by the oncologist. The amount of radiation a patient receives will depend on various considerations, but the two most important are the location of the tumor in relation to other critical structures or organs of the body, and the extent to which the tumor has spread. A typical course of treatment for a patient undergoing radiation therapy will be a treatment schedule over a 1 to 6 week period, with a total dose of between 10 and 80 Gy administered to the patient in a single daily fraction of about 1.8 to 2.0 Gy, 5 days a week. In a preferred embodiment of this invention there is synergy when tumors in human patients are treated with the combination treatment of the invention and radiation. In other words, the inhibition of tumor growth by means of the agents comprising the combination of the invention is enhanced when combined with radiation, optionally with additional chemotherapeutic or anticancer agents. Parameters of adjuvant radiation therapies are, for example, contained in WO 99/60023.

The type I anti-CD20 antibody or the afucosylated anti-CD20 antibodies are administered to a patient according to known methods, by intravenous administration as a bolus or by continuous infusion over a period of time, by intramuscular, intraperitoneal, intracerobrospinal, subcutaneous, intra-articular, intrasynovial, or intrathecal routes. In one embodiment, the administration of the antibody is intravenous or subcutaneous.

The Bcl-2 inhibitor and the MDM2 inhibitor are administered to a patient according to known methods, by intravenous administration as a bolus or by continuous infusion over a period of time, orally, by intramuscular, intraperitoneal, intracerobrospinal, subcutaneous, intra-articular, intrasynovial, or intrathecal routes. In one embodiment, the administration of said Bcl-2 inhibitor and said MDM2 inhibitor is intravenous or orally.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention is contemplated. Supplementary active compounds can also be incorporated into the compositions.

### Pharmaceutical Compositions:

Pharmaceutical compositions can be obtained by processing the anti-CD20 antibody and/or the Bcl-2 inhibitor and the MDM2 inhibitor according to this invention with pharmaceutically acceptable, inorganic or organic carriers. Lactose, corn starch or derivatives thereof, talc, stearic acids or it's salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

In one aspect of the disclosure the composition comprises both said type I anti-CD20 antibody (preferably rituximab) or said afucosylated anti-CD20 antibody with an amount of fucose is 60% or less (preferably the afucosylated humanized B-Ly1 antibody, more preferably obinutuzumab) and said Bcl-2 inhibitor and said MDM2 inhibitor for use in the treatment of cancer, in particular of CD20 expressing cancer (preferably a lymphoma or lymphocytic leukemia, more preferably a B-Cell Non-Hodgkin's lymphoma (NHL), Mantle cell lymphoma (MCL), acute lymphocytic leukemia (ALL), chronic lymphocytic leukemia (CLL), B-cell diffuse large cell lymphoma (DLCL), Burkitt's lymphoma, hairy cell leukemia, follicular lymphoma, multiple myeloma, marginal zone lymphoma, post transplant lymphoproliferative disorder (PTLD), HIV associated lymphoma, waldenstrom's macroglobulinemia, or primary CNS lymphoma).

Said pharmaceutical composition may further comprise one or more pharmaceutically acceptable carriers.

In one aspect the disclosure provides a pharmaceutical composition, e.g. for use in cancer, comprising (i) an effective first amount of a type I anti-CD20 antibody (preferably rituximab) or an afucosylated anti-CD20 antibody with an amount of fucose is 60% or less (preferably an afucosylated humanized B-Ly1 antibody), and (ii) an effective second amount of a Bcl-2 inhibitor and a MDM2 inhibitor. Such composition optionally comprises pharmaceutically acceptable carriers and / or excipients.

Pharmaceutical compositions of the type I anti-CD20 antibody or the afucosylated anti-CD20 antibody alone are prepared for storage by mixing an antibody having the desired degree of purity with optional pharmaceutically acceptable carriers, excipients or stabilizers (Remington's Pharmaceutical Sciences 16th edition, Osol, A. (ed.) (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

Pharmaceutical compositions of the Bcl-2 inhibitor and the MDM2 inhibitor can be similar to those described above for the type I anti-CD20 antibody or the afucosylated anti-CD20 antibody.

Pharmaceutical compositions of small molecule the Bcl-2 inhibitor and the MDM2 inhibitor include those suitable for oral, nasal, topical (including buccal and sublingual), rectal, vaginal and/or parenteral administration. The compositions may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of a Bcl-2 inhibitor and a MDM2 inhibitor which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent. Methods of preparing these compositions include the step of bringing into association a Bcl-2 inhibitor and a MDM2 inhibitor with the carrier and, optionally, one or more accessory ingredients. In general, the pharmaceutical compositions of the BTK inhibitor are prepared by uniformly and intimately bringing into association a Bcl-2 inhibitor and a MDM2 inhibitor with liquid carriers, or finely divided solid carriers, or both, and then, if necessary, shaping the product. Pharmaceutical compositions suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a Bcl-2 inhibitor and a MDM2 inhibitor as an active ingredient. A Bcl-2 inhibitor and a MDM2 inhibitor may also be administered as a bolus, electuary or paste.

In one aspect of the disclosure, the type I anti-CD20 antibody or the afucosylated anti-CD20 antibody and the Bcl-2 inhibitor and the MDM2 inhibitor are formulated into two separate pharmaceutical compositions.

The active ingredients may also be entrapped in microcapsules prepared, for example, by coacervation techniques or by interracial polymerization, for example, hydroxymethylcellulose or gelatin-microcapsules and poly- (methylmethacylate) microcapsules, respectively, in colloidal drug delivery systems (for example, liposomes, albumin microspheres, microemulsions, nano- particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (US 3,773,919), copolymers of L-glutamic acid and gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

Preferably, said type I anti-CD20 antibody is rituximab or said afucosylated anti-CD20 antibody is a humanized B-Ly1 antibody and said Bcl-2 inhibitor is 4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)-N-({4-[(tetrahydro-2H-pyran-4-ylmethyl)amino]-3-[(trifluoromethyl)sulfonyl]phenyl}sulfonyl)benzamide or a salt or polymorph thereof and said MDM2 inhibitor is 4-{[(2R,3S,4R,5S)-4-(4-Chloro-2-fluorophenyl)-3-(3-chloro-2-fluoro-phenyl)-4-cyano-5-(2,2-dimethyl-propyl)-pyrrolidine-2-carbonyl]-amino}-3-methoxy-benzoic acid or a salt or polymorph thereof, and said cancer is a CD20 expressing cancer, preferably a lymphoma or lymphocytic leukemia.

One aspect of the disclosure is a pharmaceutical composition comprising a combination of a type I anti-CD20 antibody or a humanized B-Ly1 antibody which is afucosylated with an amount of fucose of 60% or less of the total amount of oligosaccharides (sugars) at Asn297, and 4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)-N-({4-[(tetrahydro-2H-pyran-4-ylmethyl)amino]-3-[(trifluoromethyl)sulfonyl]phenyl} sulfonyl)benzamide or a salt or polymorph thereof and 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid or a salt or polymorph thereof, for the treatment of cancer, wherein said cancer is a CD20 expressing cancer, preferably a lymphoma or lymphocytic leukemia.

The present disclosure further provides a method for the treatment of cancer, comprising administering to a patient in need of such treatment (i) an effective first amount of a type I anti-CD20 antibody or an afucosylated anti-CD20 antibody with an amount of fucose is 60% or less, (preferably an afucosylated humanized B-Ly1 antibody); and (ii) an effective second amount of a Bcl-2 inhibitor and a MDM2 inhibitor.

In one aspect, the amount of fucose of is between 40% and 60%.

Preferably, said cancer is a CD20 expressing cancer.

Preferably, said CD20 expressing cancer is a lymphoma or lymphocytic leukemia.

Preferably, type I anti-CD20 antibody is rituximab.

Preferably, said afucosylated anti-CD20 antibody is a type II anti-CD20 antibody.

Preferably, said antibody is a humanized B-Ly1 antibody as disclosed herein.

Preferably, said antibody is obinutuzumab.

Preferably, said Bcl-2 inhibitor is 4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)-N-({4-[(tetrahydro-2H-pyran-4-ylmethyl)amino]-3-[(trifluoromethyl)sulfonyl]phenyl} sulfonyl)benzamide or a salt or polymorph thereof.

Preferably, said MDM2 inhibitor is 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino }-3-methoxy-benzoic acid or a salt or polymorph thereof. Preferably, said type I anti-CD20 antibody is rituximab or said afucosylated anti-CD20 antibody is a humanized B-Ly1 antibody and said Bcl-2 inhibitor is 4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)-N-({4-[(tetrahydro-2H-pyran-4-ylmethyl)amino]-3-[(trifluoromethyl)sulfonyl]phenyl} sulfonyl)benzamide or a salt or polymorph thereof and said MDM2 inhibitor is 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid or a salt or polymorph thereof, and said cancer is a CD20 expressing cancer, preferably a lymphoma or lymphocytic leukemia.

As used herein, the term "patient" preferably refers to a human in need of treatment with a type I anti-CD20 antibody or an afucosylated anti-CD20 antibody (e.g. a patient suffering from CD20 expressing cancer) for any purpose, and more preferably a human in need of such a treatment to treat cancer, or a precancerous condition or lesion. However, the term "patient" can also refer to non-human animals, preferably mammals such as dogs, cats, horses, cows, pigs, sheep and non-human primates, among others.

The present disclosure further provides a type I anti-CD20 antibody or an afucosylated anti-CD20 antibody with an amount of fucose is 60% or less, and a Bcl-2 inhibitor and a MDM2 inhibitor for use in the treatment of cancer.

Preferably, said type I anti-CD20 antibody is rituximab.

Preferably, said afucosylated anti-CD20 antibody is a humanized B-Ly1 antibody.

Preferably, said afucosylated humanized B-Ly1 antibody is obinutuzumab.

Preferably, said Bcl-2 inhibitor is 4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)-N-({4-[(tetrahydro-2H-pyran-4-ylmethyl)amino]-3-[(trifluoromethyl)sulfonyl]phenyl} sulfonyl)benzamide or a salt or polymorph thereof and said MDM2 inhibitor is 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid or a salt or polymorph thereof.

Preferably, said type I anti-CD20 antibody is rituximab or said afucosylated anti-CD20 antibody is a humanized B-Ly1 antibody, more preferably obinutuzumab, and said Bcl-2 inhibitor is 4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)-N-({4-[(tetrahydro-2H-pyran-4-ylmethyl)amino]-3-[(trifluoromethyl)sulfonyl]phenyl} sulfonyl)benzamide or a salt or polymorph thereof and said MDM2 inhibitor is 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid or a salt or polymorph thereof, and said cancer is a CD20 expressing cancer, preferably a lymphoma or lymphocytic leukemia.

The following examples and figures are provided to aid the understanding of the present invention, the true scope of which is set forth in the appended claims.

### Sequence Listing

- **SEQ ID NO: 1**: amino acid sequence of variable region of the heavy chain (VH) of murine monoclonal anti-CD20 antibody B-Ly1.
- **SEQ ID NO: 2**: amino acid sequence of variable region of the light chain (VL) of murine monoclonal anti-CD20 antibody B-Ly1.
- **SEQ ID NO: 3 -19**: amino acid sequences of variable region of the heavy chain (VH) of humanized B-Ly1 antibodies (B-HH2 to B-HH9, B-HL8, and B-HL10 to B-HL17)
- **SEQ ID NO: 20**: amino acid sequences of variable region of the light chain (VL) of humanized B-Ly1 antibody B-KV1

### Experimental Procedures

### Example 1:

### Example

### In vivo antitumor efficacy

The in vivo antitumor efficacy of the CD20 specific antibody obinutuzumab in combination with bcl-2 inhibitor GDC-0199 and MDM2 inhibitor RG7388 was evaluated against DOHH2 DLBCL xenografts (CD20+, p53wt). As used herein, obinutuzumab is used synonymously with RO5072759 and B-HH6-B-KV1 GE. As used herein, said bcl-2 inhibitor GDC-0199 is the following chemical compound: 4-(4-{[2-(4-chlorophenyl)-4,4-dimethylcyclohex-1-en-1-yl]methyl]piperazin-1-yl)-2-(1H-pyrrolo[2,3-b]pyridin-5-yloxy)-N-({4-[(tetrahydro-2H-pyran-4-ylmethyl)amino]-3-[(trifluoromethyl)sulfonyl]phenyl}sulfonyl)benzamide or a salt or a polymorphic form thereof. As used herein, said MDM2 inhibitor RG7388 is the following chemical compound 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid or a salt or a polymorphic form thereof.

### Test agents

CD20 antibody obinutuzumab was provided as stock solution from Roche, Basel, Switzerland. Antibody buffer included histidine. Antibody solution was diluted appropriately in buffer from stock prior injections. MDM2(4) inhibitor was provided as SDP formulation from Roche, Basel, Switzerland and resuspended prior to use. Bcl-2 inhibitor GDC-0199 was provided by GNE, SSF, USA and formulated prior to use.

### Cell line and culture conditions

The original DOHH-2 human B cell NHL cell line (DLBCL) was established at the NCI and purchased from ATCC (Manassas, VA, USA). Expansion of tumor cells for the transplantation was done by the TAP CompacT CellBase Cell Culture Roboter according to the protocol. Tumor cell line was routinely cultured in RPMI 1640 medium, FCS 10% and L-Glutamin 2 mM at 37 °C in a water-saturated atmosphere at 5 % CO₂. Culture passage was performed with trypsin / EDTA 1x splitting twice/week and passage 2 used for transplantation.

### Animals

Female SCID beige mice, age 6-7 weeks at arrival, maintained under specific-pathogen-free condition with daily cycles of 12h light /12h darkness according to committed guidelines. Experimental study protocol was reviewed and approved by local government. After arrival animals were maintained in animal facility for one week to get accustomed to new environment and for observation. Continuous health monitoring was carried out on regular basis. Diet food and autoclaved water were provided ad libitum.

### Monitoring

Animals were controlled daily for clinical symptoms and detection of adverse effects. For monitoring throughout the experiment body weight of animals was documented.

### Treatment of animals

Animal treatment started after randomisation when median tumor size was about 200mm³. CD20 antibody obinutuzumab was administered as single agent and in combination at 10mg/kg ip once weekly on days 13, 21 and 27. The corresponding vehicle was administered on the same days. MDM2(4) inhibitor RG7388 oral treatment at 30mg/kg was done as single agent and in combination on days 13-17, 20-24 and 27-29. Finally, bcl-2 inhibitor GDC-0199 was given orally at 100mg/kg on days 13-29 as single agent and in combination.

### Antitumor efficacy

DOHH-2 human Diffuse Large B-cell Lymphoma (DLBCL) cells (CD20+, p53wt) were s.c. inoculated with Matrigel onto female SCID beige mice. Tumor bearing mice were randomized 13 days later to the indicated study groups and compound treatment initiated. Tumor bearing animals were treated with vehicle control, with the MDM2(4) inhibitor RO5503781 (RG7388) at 30 mg/kg, with the anti-CD20 antibody obinutuzumab at 10mg/kg or with bcl-2 inhibitor GDC-0199 (100mg/kg) as single agent. Furthermore one study group received the triple combination of MDM2 inhibitor RG7388, CD20 antibody obinutuzumab and bcl-2 inhibitor GDC-0199. As a result all compounds given as single agent demonstrated significant anti-tumor efficacy against DOHH-2 xenografts. In more detail treatment with the MDM2(4) inhibitor RO5503781 (RG7388) resulted in 56% tumor growth inhibition against p53wt DOHH2 xenografts compared to control. A similar efficacy was noticed after treatment with the bcl-2 inhibitor GDC-0199 (60% TGI), whereas the best efficacy as single agent was achieved after treatment with the anti-CD20 antibody obinutuzumab reaching nearly tumor stasis (TGI 90%). However, superior efficacy was observed for the triple combination group including the MDM2 inhibitor RG7388 plus CD20 antibody obinutuzumab plus bcl-2 inhibitor GDC-0199. In more detail the triple combination approach substantially induced tumor regression early on which reached finally 90% with 30% complete tumor remissions. The strong efficacy of the triple combination arm was synergistically superior compared to the respective single agent arms.

**Table 2**

| **Compound** | **Dose (mg/kg)** | **Schedule** | **TGI %** | **Regression %** | **npTCR and CI** |
|---|---|---|---|---|---|
| MDM2 (4) inhibitor (RO5503781) | 30 | qdx13 | 56 | - | 0.48 (CI 0.33-**0.68**) |
| CD20 Mab (obinutuzumab) | 10 | q7dx3 | 90 | - | 0.17 (CI 0.08-**0.24**) |
| Bcl-2 inhibitor GDC-0199 | 100 | qdx17 | 60 | - | 0.43 (CI 0.27-**0.67**) |
| MDM2 (4) inhibitor+ | 30 | qdx13 | | | 0.009 (CI 0.00-**0.02**) |
| obinutuzumab+ | 10 | q7dx3 | >100 | 90 | |
| GDC-0199 | 100 | qdx17 | | | |

### Example 2

CD20 specific antibody obinuzumab in combination with bcl-2 inhibitor GDC-0199 and MDM2 inhibitor RG7388.

Also in this example, the CD20-specific antibody obinutuzumab is used in combination with bcl-2 inhibitor GDC-0199 and MDM2 inhibitor RG7388. As used herein, obinutuzumab is used synonymously with RO5072759 and B-HH6-B-KV1 GE. As used herein, said bcl-2 inhibitor GDC-0199 is the following chemical compound: 4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)-N-({4-[(tetrahydro-2H-pyran-4-ylmethyl)amino]-3-[(trifluoromethyl)sulfonyl]phenyl} sulfonyl)benzamide or a salt or a polymorphic form thereof. As used herein, said MDM2 inhibitor RG7388 is the following chemical compound 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid or a salt or a polymorphic form thereof.

### Antitumor activity of combined treatment of a type II anti-CD20 antibody RO5072759 (B-HH6-B-KV1 GE), the Bcl-2-specific inhibitor GDC-0199 (Abt-199, RG7601) and the MDM2 antagonist RG7388 (RO5503781).

### Test agents

Type II anti-CD20 antibody B-HH6-B-KV1 GE (= humanized B-Ly1, glycoengineered B-HH6-B-KV1, see WO 2005/044859 and WO 2007/031875) was provided as stock solution (c=9.4 mg/m1) from GlycArt, Schlieren, Switzerland, in histidine, trehalose and polysorbate 20 buffer. The antibody was diluted with PBS prior to in-vivo application.

The Bcl-2 inhibitor GDC-0199 was obtained from Genentech Inc., CA, USA. MDM2(4) antagonist RG7388 (RO5503781) was provided by the Small Molecule Process Research and Development unit, Hoffmann-La Roche, Basel, Switzerland.

### Cell lines and culture conditions

The human Z138 mantle cell lymphoma cell line was cultured in DMEM supplemented with 10% fetal bovine serum (PAA Laboratories, Austria) and 2 mM L-glutamine at 37°C in a water-saturated atmosphere at 5% CO2. For in-vivo xenograft experiments the cells were co-injected with Matrigel.

### Animals

Female SCID beige mice, age 4-5 weeks at arrival (purchased from Charles River, Sulzfeld, Germany), were were maintained in the quarantine part of the animal facility for one week and afterwards under specific-pathogen-free condition with daily cycles of 12 h light /12 h darkness according to guidelines (GV-Solas; Felasa; TierschG). The experimental study protocol was reviewed and approved by Roche and the local government (Regierung von Oberbayern; registration no. 55.2-1-54-2531.2-26-09). Diet food (KLIBA NAFAG 3807) and water (filtered) were provided ad libitum.

### Monitoring

Animals were monitored daily for clinical symptoms and detection of adverse effects. During the experiment the body weight of animals was checked two times a week and tumor volume was measured by caliper.

### Treatment of animals

Animal treatment started at the day of randomization 18 days after tumor cell inoculation. Humanized type II anti-CD20 antibody RO5072759 B-HH6-B-KV1 GE (obinutuzumab) was administered as single agent i.p. q7d once weekly (day 18, 25, 32) for 3 weeks at a dosage of 0.5 mg/kg. The bcl-2 inhibitor GDC-0199 was given p.o. once daily (from day 18 to day 36) over 19 days at a dosage of 100 mg/kg. The MDM2 antagonist RG7388 (RO5503781) was given p.o. once daily over 5 days at a dosage of 100 mg/kg (active compound) from day 18 to day 22 and was administered over 12 days at a dosage of 80 mg/kg (active compound) from day 25 to day 36 . There was no treatment on day 23 and 24. In the combination groups, RO5072759 B-HH6-B-KV1 GE, MDM2(4) and GDC-0199 were administered at the same dosages and on the same days. The corresponding vehicle was administered on the same days.

### Tumor growth inhibition (TGI) on day 32

Monotherapy treatment using RO5072759, GDC-0199 or RG7388 (RO5503781) resulted in tumor growth inhibition of 47 %, 53% or67%, respectively. Combination of RO5072759 with GDC-0199 or RG7388 (RO5503781) yielded a tumor growth inhibition of 85% or 86%, respectively. Combination of RG7388 (RO5503781) with GDC-0199 and the triple combination showed tumor regression (TGI>100%) on day 32 after tumor cell inoculation.

To elucidate the long term effects of the various treatments a time-to-event analysis was performed until day 125 after tumor cell inoculation. The triple combination resulted in a complete tumor remission in all animals until day 125 (study termination). Combination of RG7388 (RO5503781) and RO5072759 resulted in 5 tumor-free animals (5 out of 10). Monotherapy with RO5072759 at the suboptimal dose of 0.5 mg/kg resulted in 2 tumor-free animals at the end of the study.

The parameter "median time to event" of each group is indicated in Table 3.

**Table 3: Summary of results from the time-to-event analysis**

| Group | Animal number | * Median time to event (Days to reach TV of 1500 mm³) | Tumor-free animals on Day 125 | Number of animals with tumor regrowth | Number of animals with reduced tumor growth or non-responder |
|---|---|---|---|---|---|
| Vehicle | 10 | 25 | --- | --- | --- |
| GA101 | 10 | 28 | 2 | 0 | 8 |
| MDM2(4) | 9 | 30 | 0 | 0 | 9 |
| GDC-0199 | 10 | 29 | 0 | 0 | 10 |
| MDM2(4)+ GA101 | 10 | >125 | 5 | 2 | 3 |
| GDC-0199+ GA101 | 10 | 38 | 0 | 0 | 10 |
| GDC-0199+ MDM2(4) | 9 | 75 | 0 | 7 | 2 |
| GDC-0199+ MDM2(4)+ GA101 | 10 | not reached | 10 !!! | 0 | 0 |

| | | | | | |
|---|---|---|---|---|---|
| * The median time-to-event is the time (number of days) at which half of the animals have got an event | | | | | |

As disclosed herein and also appended in the sequence listing, the following sequences are part of the present disclosure:

### SEQUENCES

**SEQ ID NO:1**
   **amino acid sequence of variable region of the heavy chain (VH) of murine monoclonal anti-CD20 antibody B-Ly1**
**SEQ ID NO:2**
   **amino acid sequence of variable region of the light chain (VL) of murine monoclonal anti-CD20 antibody B-Ly1**
**SEQ ID NO:3**
   **amino acid sequences of variable region of the heavy chain (VH) of humanized B-Ly1 antibody (B-HH2)**
**SEQ ID NO:4**
   **amino acid sequences of variable region of the heavy chain (VH) of humanized B-Ly1 antibody (B-HH3)**
**SEQ ID NO:5**
   **amino acid sequences of variable region of the heavy chain (VH) of humanized B-Ly1 antibody (B-HH4)**
**SEQ ID NO:6**
   **amino acid sequences of variable region of the heavy chain (VH) of humanized B-Ly1 antibody (B-HH5)**
**SEQ ID NO:7**
   **amino acid sequences of variable region of the heavy chain (VH) of humanized B-Ly1 antibody (B-HH6)**
**SEQ ID NO:8**
   **amino acid sequences of variable region of the heavy chain (VH) of humanized B-Ly1 antibody (B-HH7)**
**SEQ ID NO:9**
   **amino acid sequences of variable region of the heavy chain (VH) of humanized B-Ly1 antibody (B-HH8)**
**SEQ ID NO:10**
   **amino acid sequences of variable region of the heavy chain (VH) of humanized B-Ly1 antibody (B-HH9)**
**SEQ ID NO:11**
   **amino acid sequences of variable region of the heavy chain (VH) of humanized B-Ly1 antibody (B-HL8)**
**SEQ ID NO:12**
   **amino acid sequences of variable region of the heavy chain (VH) of humanized B-Ly1 antibody (B-HL10)**
**SEQ ID NO:13**
   **amino acid sequences of variable region of the heavy chain (VH) of humanized B-Ly1 antibody (B-HL11)**
**SEQ ID NO:14**
   **amino acid sequences of variable region of the heavy chain (VH) of humanized B-Ly1 antibody (B-HL12)**
**SEQ ID NO:15**
   **amino acid sequences of variable region of the heavy chain (VH) of humanized B-Ly1 antibody (B-HL13)**
**SEQ ID NO:16**
   **amino acid sequences of variable region of the heavy chain (VH) of humanized B-Ly1 antibody (B-HL14)**
**SEQ ID NO:17**
   **amino acid sequences of variable region of the heavy chain (VH) of humanized B-Ly1 antibody (B-HL15)**
**SEQ ID NO:18**
   **amino acid sequences of variable region of the heavy chain (VH) of humanized B-Ly1 antibody (B-HL16)**
**SEQ ID NO:19**
   **amino acid sequences of variable region of the heavy chain (VH) of humanized B-Ly1 antibody (B-HL17)**
**SEQ ID NO:20**
   **amino acid sequences of variable region of the light chain (VL) of humanized B-Ly1 antibody B-KV1**

## Claims

1. A pharmaceutical composition comprising a combination of a humanized B-Ly1 antibody which is afucosylated with an amount of fucose of 60% or less of the total amount of oligosaccharides at Asn297, and 4-(4-{[2-(4-chlorophenyl)-4,4-dimethylcyclohex-1-en-1-yl]methyl]piperazin-1-yl)-N-([3-nitro-4-[(tetrahydro-2H-pyran-4-ylmethyl)amino]phenyl}sulfonyl)-2-(1H-pyrrolo[2,3-b]pyridin-5-yloxy)benzamide (GDC-0199, ABT199) and 4-1[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid (RG7388) or a salt thereof for use in the treatment of cancer.

2. The pharmaceutical composition for use according to claim 1, wherein said cancer is a CD20 expressing cancer.

3. The pharmaceutical composition for use according to claim 2, wherein said CD20 expressing cancer is a lymphoma or lymphocytic leukemia.

4. The pharmaceutical composition for use according to any one of claims 1-3, wherein the afucosylated anti-CD20 antibody is obinutuzumab.

5. The pharmaceutical composition for use according to any one of claims 1-4, wherein one or more additional other cytotoxic, chemotherapeutic or anti-cancer agents, or compounds or ionizing radiation enhancing the effects of such agents are administered.

## Patentansprüche

1. Pharmazeutische Zusammensetzung, umfassend eine Kombination aus einem humanisierten B-Ly1-Antikörper, der mit einer Menge von 60 % Fucose oder weniger der Gesamtmenge an Oligosacchariden an Asn297 afucosyliert ist, und 4-(4-{[2-(4-Chlorphenyl)-4,4-dimethylcyclohex-1-en-1-yl]methyl}piperazin-1-yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamid (GDC-0199, ABT199) und 4-{[(2R,3S,4R,5S)-4-(4-Chlor-2-fluor-phenyl)-3-(3-chlor-2-fluor-phenyl)-4-cyano-5-(2,2-dimethyl-propyl)pyrrolidin-2-carbonyl]-amino}-3-methoxy-benzoesäure (RG7388) oder einem Salz davon zur Verwendung bei der Behandlung von Krebs.

2. Pharmazeutische Zusammensetzung zur Verwendung nach Anspruch 1, wobei der Krebs ein CD20-exprimierender Krebs ist.

3. Pharmazeutische Zusammensetzung zur Verwendung nach Anspruch 2, wobei der CD20-exprimierende Krebs ein Lymphom oder lymphozytische Leukämie ist.

4. Pharmazeutische Zusammensetzung zur Verwendung nach einem der Ansprüche 1-3, wobei der afucosylierte Anti-CD20-Antikörper Obinutuzumab ist.

5. Pharmazeutische Zusammensetzung zur Verwendung nach einem der Ansprüche 1-4, wobei ein oder mehrere zusätzliche andere zytotoxische, chemotherapeutische oder Antikrebsmittel oder Verbindungen oder ionisierende Strahlung, die die Wirkungen solcher Mittel verstärken, verabreicht werden.

## Revendications

1. Composition pharmaceutique comprenant une combinaison d'un anticorps B-Ly1 humanisé qui est afucosylé avec une quantité de fucose inférieure ou égale à 60 % de la quantité totale d'oligosaccharides au niveau de Asn297, et de 4-(4-{[2-(4-chlorophényl)-4,4-diméthylcyclohex-1-én-1-yl]méthyl}pipérazin-1-yl)-N-({3-nitro-4-[(tétrahydro-2H-pyran-4-ylméthyl)amino]phényl}sulfonyl)-2-(1H-pyrrolo[2,3-b]pyridin-5-yloxy)benzamide (GDC-0199, ABT199) et d'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque (RG7388) ou d'un sel de celui-ci pour une utilisation dans le traitement d'un cancer.

2. Composition pharmaceutique pour une utilisation selon la revendication 1, dans laquelle ledit cancer est un cancer exprimant le CD20.

3. Composition pharmaceutique pour une utilisation selon la revendication 2, dans laquelle ledit cancer exprimant le CD20 est un lymphome ou une leucémie lymphoïde.

4. Composition pharmaceutique pour une utilisation selon l'une quelconque des revendications 1 à 3, dans laquelle l'anticorps anti-CD20 afucosylé est l'obinutuzumab.

5. Composition pharmaceutique pour une utilisation selon l'une quelconque des revendications 1 à 4, dans laquelle sont administrés un ou plusieurs autres agents cytotoxiques, chimiothérapeutiques ou anti-cancéreux supplémentaires, ou des composés ou un rayonnement ionisant améliorant les effets de tels agents.
